# EUROPÄISCHE PATENTSCHRIFT

(11) **EP 2 621 455 B1**
(45) Veröffentlichungstag und Bekanntmachung des Hinweises auf die Patenterteilung: **26.06.2019**
(21) Anmeldenummer: 11767209.7
(22) Anmeldetag: 23.09.2011
(51) Int. Cl.: A61K 6/00, A61K 6/083

(54) **POLYMERISIERBARES DENTALMATERIAL MIT REAKTIV-PASTENBILDNER, GEHÄRTETES DENTALMATERIAL UND DEREN VERWENDUNG**
POLYMERIZABLE DENTAL MATERIAL COMPRISING A REACTIVE PASTE FORMER, HARDENED DENTAL MATERIAL AND USE THEREOF
MATÉRIAU DENTAIRE POLYMÉRISABLE CONTENANT UN AGENT RÉACTIF DE FORMATION DE PÂTE, MATÉRIAU DENTAIRE DURCI, ET LEUR UTILISATION

(30) Priorität: 28.09.2010 DE 102010046697
(43) Veröffentlichungstag der Anmeldung: 07.08.2013
(73) Patentinhaber: Kettenbach GmbH & CO. KG, 35713 Eschenburg (DE)
(72) Erfinder: BUBLEWITZ, Alexander, 35745 Herborn (DE); THEIS, Alexander, 35713 Eschenburg (DE); REBER, Jens-Peter, 58540 Meinerzhagen (DE)
(74) Vertreter: Keil & Schaafhausen Patent- und Rechtsanwälte PartGmbB
(86) Internationale Anmeldenummer: PCT/EP2011/066557
(87) Internationale Veröffentlichungsnummer: WO 2012/052249

(56) Entgegenhaltungen:
- EP-A1- 2 198 824
- US-A- 3 347 954
- US-A1- 2009 192 239
- US-B1- 6 852 775

## Beschreibung

Die vorliegende Erfindung betrifft ein polymerisierbares Dentalmaterial in Form einer pastösen Mehrkomponentenformulierung enthaltend wenigstens einen monomeren, oligomeren und/oder polymeren Reaktiv-Pastenbildner, mono-, bi- oder oligo- bis polyfunktionelle Acrylsäureester und/oder Methacrylsäureester (nachstehend gemeinschaftlich "(Meth)acrylsäureester" genannt) und Barbitursäurederivate und/oder Malonylsulfamide. Die Erfindung bezieht sich zudem auf ein polymerisiertes Dentalmaterial sowie auf die Verwendung des polymerisierbaren und des polymerisierten Dentalmaterials beispielsweise als Kronen- und Brückenmaterial.

Solche Materialien dienen beispielsweise zur Herstellung von mehrgliedrigen Brücken, Kronen, Inlays, Onlays oder Stiftprovisorien nach endodontischer Behandlung.

In der Prothetik kennt man eine direkte oder indirekte Methode zur Anfertigung von Provisorien. Unter der direkten Methode versteht man die Herstellung der Provisorien durch den Zahnarzt unmittelbar nach der Präparation. Die indirekte Herstellung erfolgt über die Anfertigung eines Modells im Labor.

So werden z.B. provisorische Kronen- und Brückenmaterialien in der Zahnmedizin zur Abdeckung der Dentinwunde nach erfolgten Präparationen oder auch zur temporären Versorgung von Implantataufbauten hergestellt. Bei der Versorgung natürlicher Zahnstümpfe dienen sie dem Schutz der Pulpa vor thermischen, chemischen und bakteriellen Einflüssen. Weitere Aufgaben liegen in der Aufrechterhaltung der Kaufunktion, der Fixierung der okklusalen bzw. sagittalen Kieferrelation und der Verhinderung des Einwachsens der benachbarten Gingiva auf den Zahnstumpf.

Vor der Präparation des betreffenden Zahns wird von dem entsprechenden Kieferabschnitt eine Abformung genommen, durch welche die ursprüngliche Situation und die Form des zu präparierenden Zahns festgehalten werden. Nach der Präparation wird die Impression des beschliffenen Zahns in der Abformung mit dem provisorischen Kronen- und Brückenmaterial ausgefüllt. Der so vorbereitete Abdruck wird auf den Kiefer reponiert. Hat das provisorische Kronen- und Brückenmaterial einen gummielastischen Zustand erreicht, kann die Abformung mit dem geformten, rohen Provisorium entnommen und nach vollständiger Aushärtung bearbeitet, auf den Zahnstumpf angepasst und die Okklusion (Kaufläche) eingeschliffen werden. Das Einsetzen des Provisoriums erfolgt mit speziellen provisorischen Befestigungszementen.

Gattungsgemäße polymerisierbare Dentalmaterialien sind aus dem Stand der Technik an sich bekannt. Sie enthalten polymerisierbare Monomere, deren Polymerisation durch gebildete Radikale ausgelöst wird. Diese Radikale werden in der Regel durch die Reaktion eines geeigneten Initiators mit einem Coinitiator gebildet, wobei die Radikalbildung unmittelbar nach deren Zusammenbringung beginnt. Der Initiator, der bei Raumtemperatur alleine eine ausreichende Lagerstabilität besitzt, muss daher zur Lagerung der Dentalmaterialien vom Coinitiator getrennt aufbewahrt werden. Folglich werden meistens MehrkomponentenSysteme eingesetzt, deren Komponenten erst direkt vor der Verarbeitung des Dentalmaterials miteinander in Kontakt gebracht und sorgfältig miteinander vermischt werden.

Ein aus dem Stand der Technik für gattungsgemäße Dentalmaterialien bekanntes Initiatorsystem weist ein zumeist aromatisches Amin in Kombination mit einem organischen Peroxid auf, wie es beispielsweise in DE 975 072 beschrieben ist, wobei die Redoxreaktion zwischen Amin und Peroxid die Radikale liefert. Solche aminbasierten Initiatorsysteme weisen jedoch wesentliche Nachteile auf, insbesondere toxische und/oder allergieauslösende Wirkungen der Komponenten und deren Folgeprodukte und eine allgemein schlechte Farbstabilität. Diese und weitere Nachteile sind detailliert in EP 1 872 767 A1 beschrieben.

In dieser Veröffentlichung werden zudem bei der Würdigung des Standes der Technik als Alternativen zu den bekannten Initiatorsystemen CH-acide Verbindungen, insbesondere Barbitursäurederivate, in Kombination mit Übergangsmetallionen und Chloridionen genannt. Dieses alternative Initiatorsystem besitzt eine günstigere Temperaturentwicklung bei der Polymerisation und die erhaltenen Polymerisate weisen eine deutlich bessere Farbstabilität auf.

Bei dem auf Barbitursäure bzw. deren Derivaten basierenden Initiatorsystem müssen die Barbitursäure(derivate) von den polymerisierbaren Monomeren getrennt aufbewahrt werden. Dies liegt darin begründet, dass CH-acide Verbindungen, wie die Derivate der Barbitursäure, bereits ohne die Mitwirkung von Cu(II)- und Chlorid-Ionen durch Autoxidation durch Luftsauerstoff Hydroperoxide bilden. Diese Hydroperoxide zerfallen unter Bildung von Radikalen, welche die Polymerisation der reaktiven Monomere initiieren, so dass es binnen kurzer Zeit zur spontanen Polymerisation kommt. Dieser spontane Polymerisationsprozess kann durch Zugabe von Stabilisatoren für kurze Zeit (im Bereich von wenigen Stunden) verzögert oder unterdrückt werden, nicht hingegen über einen längeren Zeitraum, wie es bei lagerstabilen Systemen erwünscht ist.

Relativ einfache Dentalformulierungen mit Redox-Initiatorsystemen, welche Barbitursäure(derivate) als Initiatorkoponente enthalten, sind aus den DE 101 24 029 A1 und DE 38 06 740 C1 bekannt. Beschrieben werden dort Pulver/Flüssigkeit-Systeme, bei denen Barbitursäure(derivate) in Pulverform in die flüssige Monomermischung eingerührt werden. Da dies jedoch über eine Handanmischung erfolgt, sind diese Systeme vergleichsweise schwierig zu dosieren und Luftblasen sind in der fertigen Krone oder Brücke nur sehr schwer oder gar nicht zu vermeiden.

Solche Probleme werden gemäß des Standes der Technik durch den Einsatz von sogenannten Paste/Paste Systemen vermieden. Dabei werden Barbitursäure(derivate) in einer Paste, bestehend aus inerten Weichmachern und Füllstoffen dispergiert. Dabei ist es erforderlich, dass in der den Initiator enthaltenden Komponente ausschließlich Verbindungen, die mit CH-aciden Verbindungen nicht vorzeitig polymerisieren enthalten sind, d.h. man verwendet in der Initiatorkomponente im wesentlichen nicht polymerisierende Verbindungen, die keine Doppelbindungen enthalten.

Die EP 1 194 110 B1 offenbart gattungsgemäße polymerisierbare Dentalmassen, die mindestens ein bi- oder höherfunktionelles ethylenisch ungesättigtes Monomer, mindestens ein monofunktionelles ethylenisch ungesättigtes Monomer, einen Beschleuniger, ein Redoxinitiatorsystem, das die adikalische Polymerisation auslösen kann, Füllstoffe, Thixotropie-Hilfsmittel, Verzögerer und andere Hilfsstoffe und einen üblichen Weichmacher enthält, wobei das Redoxinitiatorsystem ein Barbitursäurederivat und/oder ein Malonylsulfamid und ein aus der Gruppe der ein- oder mehrfunktionellen Carbonsäureperoxyester ausgewähltes organisches Peroxid umfasst.

Auch die polymerisierte Dentalmasse der EP 1 194 110 B1 weist einen gewissen Anteil an unreagierten Bestandteilen auf, die nicht in die polymerisierte Dentalmasse eingebunden sind. Dabei handelt es sich um übliche im Dentalbereich verwendete Weichmacher. Diese sind gegenüber einer Polymerisation inert, d.h. sie enthalten keine radikalisch polymerisierbaren Doppelbindungen und werden deshalb nicht einpolymerisiert. Aus diesem Grund sind die mechanischen Eigenschaften des auf diese Weise erhaltenen Polymerisates verbesserungsbedürftig. Darüber hinaus können im aggressiven Regime des Mundes solche unreagierten Bestandteile herausgelöst werden und gesundheitsschädliche Reaktionen hervorrufen.

Der in der EP 1 872 767 A1 beschriebenen Erfindung lag die Aufgabe zu Grunde, ein Initiatorsystem für polymerisierbare Dentalmaterialien zu schaffen, das die aus dem Stand der Technik bekannten Nachteile vermeidet, und darüber hinaus auch in 1:1 dosierenden Materialien wie Füllungs- und Verblendkunststoffen einsetzbar ist.

Gemäß EP 1 872 767 A1 wurde im Unterschied zu den Startersystemen basierend auf CH-aciden Verbindungen aus dem Stand der Technik eine Vorstufe des aktiven Startermoleküls eingesetzt, nämlich ein Salz der CH-aciden Verbindung. Die CH-acide Verbindung wird erst nach der Zugabe einer Säure, deren Säurestärke größer ist als die der als Salz vorliegenden CH-aciden Verbindung, freigesetzt und kann anschließend als Startermolekül für den Polymerisationsprozess der Monomere fungieren. Gemäß EP 1 872 767 A1 wurde erkannt, dass im Unterschied zu den CH-aciden Verbindungen des Standes der Technik Salze der CH-aciden Verbindung auch über längere Zeiträume lagerstabil sind. Damit wird die Initiatoraktivität für die Polymerreaktion der polymerisierbaren Monomere auch bei längerer Lagerung der Komponenten des polymerisierbaren Dentalmaterials sichergestellt. Gemäß diesem Dokument sollen Pasten, aus denen das polymerisierbare Dentalmaterial angemischt wird, und insbesondere die das Salz der CH-aciden Verbindung enthaltende Paste, besonders bevorzugt mehr als 24 Monate farb- und/oder lagerstabil sein. Das Problem mit derartigen Formulierungen ist, dass das Freisetzen der katalytisch aktiven freien Säure der CH-aciden Verbindung nur langsam vorangeht und dass damit die Aushärtereaktionen vergleichsweise lange dauern.
Aus EP 1 881 010 A1 sowie aus EP 2 239 275 A1 sind polymerisierbare Zusammensetzungen bekannt, bei denen Salze von Barbitursäurederivaten und polymerisierbare Monomere ohne Säurefunktionalität in einer Initiatorkomponente enthalten sind bzw. bei denen ein Redoxinitiatorsystem enthaltend ein Salze eines Barbitursäurederivats, eine saure Komponente oder einen Vorläufer davon sowie eine Kupferverbindung bereit gestellt werden. Es werden verschiedene Formulierungen beschrieben, darunter auch Paste/Paste-Kombinationen. Da Mischungen von Barbitursäure bzw. von Barbitursäurederivaten mit polymerisierbaren ethylenisch ungesättigten Verbindungen nicht lagerstabil sind, wird hier ein Salz der Barbitursäure bzw. ein Salz eines Barbitursäurederivats eingesetzt. Auch hier erfolgt das Auslösen der Aushärtereaktion durch eine Kombination des Salzes der Barbitursäure bzw. des Barbitursäurederivats mit der sauren Verbindung, wodurch die katalytisch aktive freie Säure der CH-aciden Verbindung nur vergleichsweise langsam freigesetzt wird.

Aus EP 2 198 824 A1 ist ein polymerisierbares Dentalmaterial bekannt. Dieses wird in wenigstens zwei Komponenten A und B formuliert, wobei Komponente A neben einem radikalisch polymerisierbaren Monomer ohne Säuregruppe ein spezielles Peroxid enthält und Komponente B ein radikalisch polymerisierbares Monomer ohne Säuregruppe und ein Salz einer CH-aziden Verbindung, die als CH-azide Verbindung eine radikalische Polymerisation auslösen kann, enthält. Auch bei diesem polymerisierbaren Dentalmaterial wird also eine Vorstufe des aktiven CH-aciden Startermoleküls eingesetzt, nämlich ein Salz dieser CH-aciden Verbindung. Die Kombination Salz einer CH-aziden Verbindung mit einem ausgewählten ethylenisch ungesättigten Monomer in Komponente B gestattet eine ausreichend lange Lagerstabilität. Das Salz der CH-aciden Verbindung, z.B. ein Salz der Barbitursäure oder ein Salz eines Barbitursäurederivats wird erst nach der Kombination mit dem Peroxid aus der Komponente A aktiviert und bewirkt sodann eine Polymerisation der vorhandenen Monomeren. Diese Stoffzusammensetzung lässt sich bei Raumtemperatur gut polymerisieren, ohne dass eine Säure zugegen ist. Das Material zeigt ein gutes Erhärtungsverhalten und gute mechanische Eigenschaften.

In der prioritätsälteren WO 2011/083020 A2 und der dazu parallelen DE 10 2009 058 638 A1 werden Mehrkomponentensysteme zur Herstellung eines Dentalmaterials beschrieben. Eine Komponente enthält wenigstens ein radikalisch polymerisierbares Harz und wenigstens einen Polymerisationsbeschleuniger und die zweite Komponente enthält eine bei Raumtemperatur flüssige oder pastöse inerte Matrix und/oder wenigstens ein radikalisch polymerisierbares Harz sowie wenigstens eine CH-azide Verbindung oder ein Salz davon als Polymerisationsinitiator. In einer oder beiden Komponenten enthält dieses System einen Perester, ein Peractetal oder ein Perketal. Für den Fall, dass in der zweiten Komponente (also in der den Polymerisationsinitiator enthaltenden Komponente) ein radikalisch polymerisierbares Harz vorgesehen ist wird in diesem Dokument gefordert, dass der Polymerisationsinitiator ein Salz einer CH-aziden Verbindung sein muss. Auch bei diesem polymerisierbaren Dentalmaterial gestattet nur die Kombination des Salzes einer CH-aziden Verbindung mit einem radikalisch polymerisierbaren Harz in der zweiten Komponente eine ausreichend lange Lagerstabilität. Das Salz der CH-aciden Verbindung, z.B. ein Salz der Barbitursäure oder ein Salz eines Barbitursäurederivats wird erst nach der Kombination beider Komponenten aktiviert und bewirkt sodann eine Polymerisation der vorhandenen Monomeren.

Daraus wird deutlich, dass Initiatorsysteme basierend auf Barbitursäurederivaten bzw. Malonylsulfamiden und ionogen gebundenem Halogen und einer Schwermetallverbindung hinsichtlich ihrer Reaktionskinetik und der mechanischen Eigenschaften nach der Polymerisation verbesserungswürdig sind. Es wird angenommen, dass diese Problematik durch das Vorhandensein von inerten, nicht reaktiven Pastenbildern maßgeblich beeinflusst wird.

Insbesondere sind für den Einsatz im Dentalbereich Materialien mit besonders hoher Festigkeit wünschenswert. Beispielsweise müssen Dentalmassen zur Erstellung von Kronen und Brücken sowohl sehr bruchstabil sein als auch die Relation der Zahnstümpfe ausreichend stabilisieren.

Aufgabe der vorliegenden Erfindung ist es, ein polymerisierbares pastenförmiges 2-Komponenten-Dentalmaterial bereitzustellen, mit dem die aus dem Stand der Technik bekannten Nachteile vermieden werden und gleichzeitig eine ausgezeichnete Lagerstabilität sowie reproduzierbare und ausreichend kurze Aushärtezeiten unter Mundbedingungen gewährleistet sind. Insbesondere lassen sich solche Dentalmaterialien zu Polymerisaten mit ausgezeichneten mechanischen Eigenschaften verarbeiten.

Diese Aufgabe wird bei dem erfindungsgemäßen polymerisierbaren Dentalmaterial dadurch gelöst, dass es in Form einer Mehrkomponenten-formulierung, insbesondere einer Zweikomponentenformulierung aus den Komponenten A und B bereitgestellt wird, worin die einzelnen Komponenten in Pastenform vorliegen und ausgewählte Inhaltsstoffe enthalten. So wird in Komponente A unter anderem ein reaktiver Pastenbildner a) mit Maleinsäure- und/oder FumarsäureEinheiten und/oder ein reaktiver Pastenbildner b) mit endständigen und/oder seitenständigen Allyl- und/oder Methallylgruppen eingesetzt.

Als Grundlage der vorliegenden Erfindung wurde erkannt, dass eine der Ursachen für die Nachteile des Standes der Technik auf den verwendeten inerten, d.h. nicht reaktiven Pastenbildnern beruht, die keine ungesättigten Kohlenstoff-Kohlenstoff-Bindungen enthalten.

Überraschenderweise wurde gefunden, dass ausgewählte Alkenylgruppen enthaltende Reaktiv-Pastenbildner bei Raumtemperatur und bei Stresstemperatur von 40°C bzw. 60°C lagerstabil mit Barbitursäurederivaten und/oder mit Malonylsulfonamiden als Initiatorpaste A formuliert werden können, ohne dass eine vorzeitige Polymerisation der ausgewählten Alkenylgruppen stattfindet. Darüber hinaus wurde überraschenderweise gefunden, dass in der lagerstabilen Katalysatorpaste A enthaltene Barbitursäurederivate und/oder Malonylsulfonamide sowie Alkenylgruppen enthaltende Reaktiv-Pastenbildner nach dem Vermischen mit der Basispaste B zusammen mit den darin enthaltenen polymerisierbaren (Meth)acrylatgruppen enthaltenden Verbindungen in einer radikalischen Polymerisation unter Mundtemperatur (∼35°C) copolymerisieren und dass dabei die Alkenylgruppen enthaltenden Reaktiv-Pastenbildner sowie die (Meth)acrylatgruppen enthaltenden Verbindungen in das entstehende Netzwerk mit eingebunden werden. Dadurch werden im Vergleich mit den Formulierungen des Standes der Technik, die nicht reaktive Pastenbildner enthalten, nach der Polymerisation im Endprodukt signifikant bessere mechanische Endeigenschaften erzielt. Dies wird z.B. durch höhere Biegefestigkeiten und E-Module im Dreipunktbiegeversuch dokumentiert.

Offenbar können ausgewählte Alkenylgruppen enthaltende Verbindungen durch die CH-aciden Barbitursäurederivate und/oder Malonylsulfenamide während der Lagerung in Abwesenheit der Co-Initiatoren (Metallionen wie z.B. Cu- Ionen und Halogenid-Ionen) nicht polymerisiert werden, wie dies bei (Meth)acrylaten und Vinylethern der Fall ist (vergl. z.B. DE 10 017 188 B4). Andererseits werden diese ausgewählten Alkenylverbindungen bei der Polymerisation der (Meth)acrylatmonomere unter Mundtemperatur als Comonomere mit einpolymerisiert und führen zu besseren mechanischen Eigenschaften als bei den aus dem Stand der Technik bekannten Massen. Ein weiterer Vorteil ist, dass durch das Einbinden der ausgewählte Alkenylgruppen enthaltenden Reaktiv-Pastenbildner im Vergleich zu den nach dem Stand der Technik eingesetzten alkenylfreien, nicht reaktiven Pastenbildnern, z.B. 2,2 Bis-4-(2-hydroxyethoxyphenyl)propanbisacetat (DE 10 017 188), Polyethylenglycole (EP 0 563 749 A1), Phtalate, Polyester der Anteil der herauslösbaren Verbindungen geringer wird und damit eine hohe Biokompatibilität zu erwarten ist.

Die vorliegende Erfindung betrifft daher ein polymerisierbares Dentalmaterial enthaltend wenigstens eine pastenförmige Komponente A und wenigstens eine pastenförmige Komponente B, wobei Komponente A mindestens einen Initiator der radikalischen Polymerisation c) ausgewählt aus der Gruppe der Barbitursäurederivate und/oder der Malonylsulfamide enthält und wobei Komponente B mindestens eine Acrylsäureester- und/oder Methacrylsäureesterreste umfassende organische Verbindung d), mindestens eine Metallverbindung e) und mindestens eine Halogenid- und/oder Pseudohalogenidverbindung f) enthält und wobei das polymerisierbare Dentalmaterial dadurch gekennzeichnet ist, dass Komponente A als Reaktiv-Pastenbildner mindestens eine von Maleinsäure und/oder von Fumarsäure abgeleitete organische Verbindung a), die neben den von Maleinsäure und/oder von Fumarsäure abgeleiteten Gruppen keine weiteren ethylenisch ungesättigten Gruppen aufweist, und/oder mindestens eine Verbindung b) umfassend mindestens einen Allyl- und/oder Methallylrest und gegebenenfalls von Maleinsäure und/oder Fumarsäure abgeleitete Einheiten, die neben den vorstehend genannten ethylenisch ungesättigten Gruppen keine weiteren ethylenisch ungesättigten Gruppen aufweist, enthält.

Bei Inhaltsstoff a) kann es sich um beliebige von Maleinsäure und/oder von Fumarsäure abgeleitete organische Verbindungen handeln. Diese Verbindungen weisen neben den vorstehend genannten ethylenisch ungesättigten Resten keine weiteren ethylenisch ungesättigten Reste auf, wie z.B. Vinylethergruppen und/oder (Meth)acrylatgruppen.

Bei den von Maleinsäure und/oder von Fumarsäure abgeleiteten organischen Verbindungen kann es sich um monomere, oligomere oder polymere Verbindungen handeln. Beispiele für monomere Verbindungen sind Maleinsäure, Fumarsäure und deren Derivate, wie z.B. deren Mono- oder Diester, Mono- oder Diamide oder Anhydride. Beispiele für solche Derivate sind Mono- oder Dialkylester sowie Fumarsäure- oder Maleinsäureanhydrid.

Erfindungsgemäß eingesetzt werden polymerisierbare Dentalmaterialien enthaltend wenigstens eine pastenförmige Komponente A und wenigstens eine pastenförmige Komponente B mit den oben genannten Inhaltsstoffen a) bis f) in der oben genannten Verteilung auf die Komponenten A und B, wobei Komponente A als Reaktiv-Pastenbildner mindestens eine von Maleinsäure und/oder von Fumarsäure abgeleitete organische Verbindung a), die neben den von Maleinsäure und/oder von Fumarsäure abgeleiteten Gruppen keine weiteren ethylenisch ungesättigten Gruppen aufweist, und/oder mindestens eine Verbindung b) umfassend mindestens einen Allyl- und/oder Methallylrest und gegebenenfalls von Maleinsäure und/oder Fumarsäure abgeleitete Einheiten, die neben den vorstehend genannten ethylenisch ungesättigten Gruppen keine weiteren ethylenisch ungesättigten Gruppen aufweist, enthält.

Vorzugsweise weisen diese Verbindungen Gruppen von Maleinsäure- und/oder Fumarsäurediamiden und ganz besonders bevorzugt von Maleinsäure- und/oder des Fumarsäurediestern auf. Besonders bevorzugt handelt es sich dabei um organische Verbindungen der Formeln Ia, Ib, IIa, IIb und insbesondere der Formeln IIc oder IId 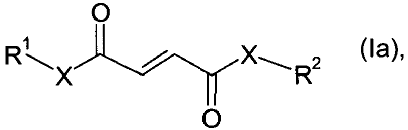 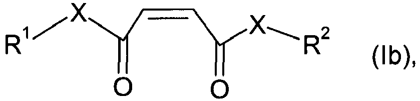 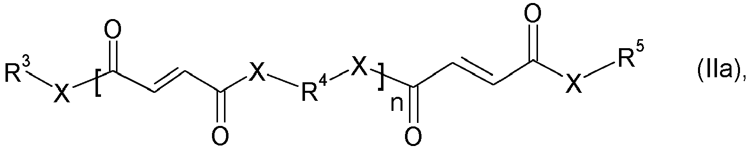 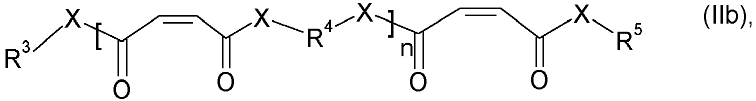 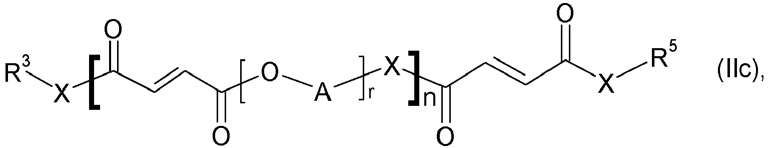 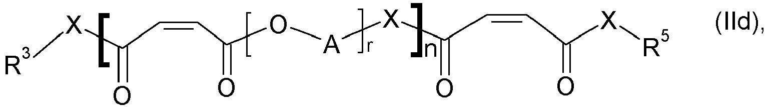
worin X Sauerstoff oder eine Gruppe -NR⁶- bedeutet,
R¹, R², R³ und R⁵ unabhängig voneinander Wasserstoff, Alkyl, Cycloalkyl, Aryl, Aralkyl oder Heterocyclyl bedeuten, die gegebenenfalls einen oder mehrere Substituenten aufweisen,
R⁴ Alkylen, Alkylenglykolether, Cycloalkylen, Arylen, Aralkylen oder Heterocyclylen bedeutet, das gegebenenfalls einen oder mehrere Substituenten aufweist,
A = CH₂-CH₂, CH₂-CH(CH₃) oder CH₂-CH₂-CH₂-CH₂ bedeutet,
R⁶ Wasserstoff, Alkyl, Cycloalkyl, Aryl, Aralkyl oder Heterocyclyl ist,
n eine ganze Zahl von 1 bis 50 ist und
r eine ganze Zahl von 1 bis 100 bedeutet.

Ein oder mehrere der oben genannten Reste R¹ bis R⁵ können gegebenenfalls substituiert sein. Beispiele für Substituenten sind weiter unten beschrieben.

In einer Ausführungsform weisen ein oder mehrere der oben genannten Reste R¹ bis R⁵ Substituenten mit Säurefunktionen auf, wie z.B. Phosphorsäuregruppen, Phosphonsäuregruppen, Sulfonsäuregruppen und/oder Carbonsäuregruppen sowie deren Anhydride. Dentalmassen mit solchen freie Säuregruppen enthaltenden Verbindungen eignen sich insbesondere zur Verwendung als selbstätzende Dentalzemente.

In einer weiteren Ausführungsform weisen ein oder mehrere der oben genannten Reste R¹ bis R⁵ neben den Substituenten mit Säurefunktionen auch Hydroxylgruppen als Substituenten auf und/oder es werden Gemische von säure- und hydroxyfunktionalisierten Verbindungen der Formeln Ia, Ib, IIa, IIb, IIc und/oder IId eingesetzt. Solche erfindungsgemäß bevorzugt eingesetzte Reaktiv-Pastenbildner a) können beispielsweise nicht umgesetzte freie Carboxyl- und/oder Hydroxylendgruppen aufweisen und/oder solche Gruppen, bei denen freie Säure- und/oder Hydroxyfunktionen durch Funktionalisierung mit sauren Verbindungen modifiziert worden sind, z.B. durch Umsetzung mit Phosphorpentoxid.

In der Praxis werden die Verbindungen der Formeln IIa, IIb, IIc und IId häufig als statistische Gemische von Malein- und Fumarsäureestern oder -amiden sowie von Polyestern oder Polyamiden unterschiedlicher Kettenlänge vorliegen. Darüber hinaus können auch Mischformen der Verbindungen der Formeln IIa und IIb bzw. IIc und IId auftreten, in denen in einem Molekül sowohl Einheiten der Fumarsäure als auch der Maleinsäure vorhanden sind.

Bevorzugte Komponenten a) sind Gemische von zwei oder mehreren der Formeln Ia, Ib, IIa, IIb, IIc und IId und/oder Gemische von Verbindungen, bei denen der Index n und der Index r im Rahmen der gegebenen Definitionen unterschiedliche Werte annehmen.

Beispiele für besonders bevorzugte erfindungsgemäß eingesetzte Reaktiv-Pastenbildner a) sind:

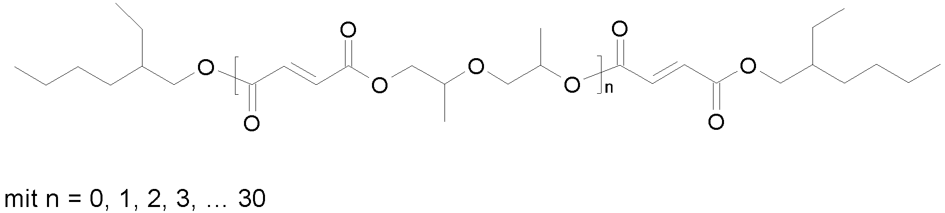

Alternativ zu der oben dargestellten Verbindung mit Di-propylenglykol-Einheiten [-O-CH₂-CH(CH₃)-]₂ können auch vorzugsweise Verbindungen mit Propylenglykol-Einheiten [-O-CH₂-CH(CH₃)-]ᵣ oder mit Ethylenglykoleinheiten [-O-CH₂-CH₂-]ᵣ oder mit Butylenglykol-, wie Tetramethylenglykoleinheiten, [-O-CH₂-CH₂-CH₂-CH₂-]ᵣ oder mit Cyclopentyleneinheiten [-O-C₅H₈-]ᵣ oder mit Cyclohexyleneinheiten [-O-C₆H₁₀-]ᵣ oder mit Phenyleneinheiten [-O-C₆H₄-]ᵣ oder mit Naphthyleneinheiten [-O-C₁₀H₆-]ᵣ oder mit Benzyleneinheiten [-O-CH₂-C₆H₄-]ᵣ eingesetzt werden. Der Index r kann dabei beliebige ganzzahlige Werte von 1 bis 100 annehmen.

Alternativ zu der oben dargestellten Verbindung mit 2-Ethylhexanoleinheiten am Kettenende können auch vorzugsweise andere einwertige Alkohole wie z.B. Benzylalkohol oder Ethanol als Endgruppe eingesetzt werden.

Weitere Beispiele für ganz besonders bevorzugte erfindungsgemäß eingesetzte Reaktiv-Pastenbildner a) mit Säurefunktion sind:

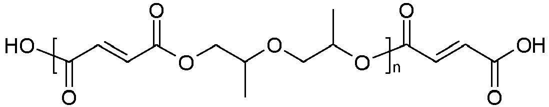

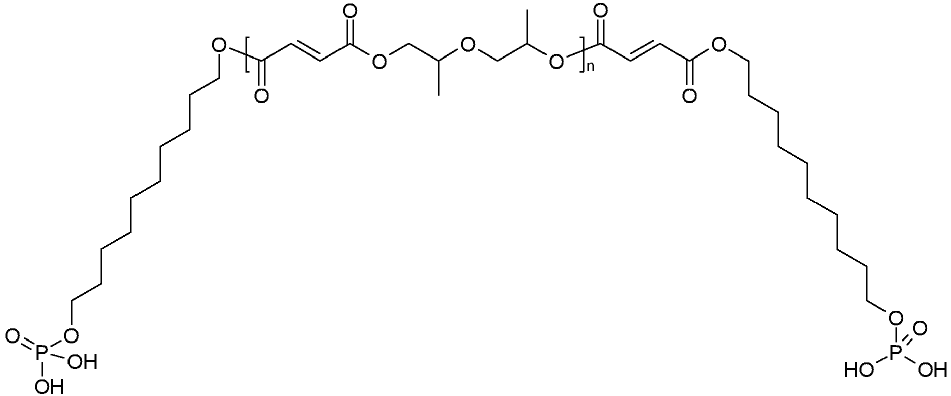

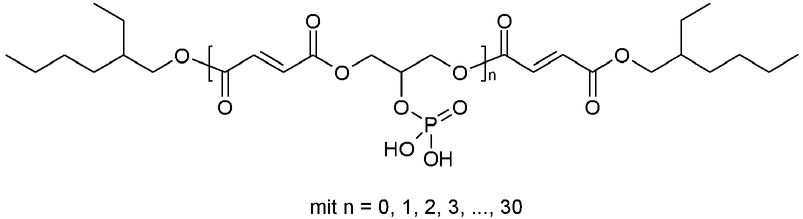

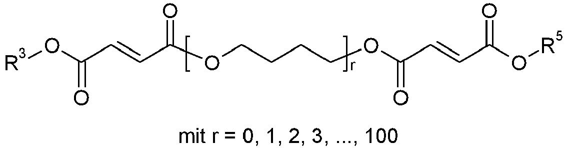

Wobei R³ und R⁵ unabhängig voneinander Wasserstoff, Alkyl, Cycloalkyl, Aryl, Aralkyl oder Heterocyclyl bedeuten, die gegebenenfalls einen oder mehrere Substituenten aufweisen, mit der Massgabe, dass mindestens einer der Reste R³ oder R⁵ Wasserstoff bedeutet.

Bei Inhaltsstoff b) kann es sich um beliebige Verbindungen mit mindestens einem Allyl- und/oder Methallylrest handeln, die gegebenenfalls noch von Fumarsäure und/oder von Maleinsäure abgeleitete Einheiten aufweisen können. Dabei sind neben organischen auch anorganische Verbindungen möglich. Diese Verbindungen weisen neben den vorstehend genannten ethylenisch ungesättigten Resten keine weiteren ethylenisch ungesättigten Reste auf, wie z.B. Vinylethergruppen und/oder (Meth)acrylatgruppen. Salze enthaltend Allyl- und/oder Methallylreste sind als Komponente b) ausgeschlossen.

Bei den Allyl- und/oder Methallylresten in den Verbindungen des Inhaltsstoffes b) handelt es sich bevorzugt um Allylether- und/oder um Methallyletherreste.

Besonders bevorzugt handelt es sich bei Inhaltsstoff b) um Verbindungen der Formel III

R⁷-[-Y-R⁸]ₘ (III),

worin R⁷ ein m-wertiger Rest ist, der gegebenenfalls einen oder mehrere Substituenten aufweist,
R⁸ eine Gruppe -CH₂-CR⁹=CH₂ ist,
R⁹ Wasserstoff oder Methyl bedeutet,
m eine ganze Zahl von 1 bis 12, bevorzugt 1 bis 8, besonders bevorzugt 1 bis 6, und ganz besonders bevorzugt 1 bis 4 ist, und
Y ausgewählt wird aus der Gruppe der kovalenten Bindung oder einem zweiwertigen Rest.

Verbindungen der Formel III können als Einzelverbindungen oder als Gemische unterschiedlicher Verbindungen der Formel III eingesetzt werden. Insbesondere bei höherfunktionellen Verbindungen mit drei oder mehr (Meth)allylgruppen können Gemische aus unterschiedlichen Verbindungen der Formel III mit unterschiedlichem Funktionalisierungsgrad eingesetzt werden. Ein Beispiel_hierfür sind technische Gemische von (Meth)allylpentaerythritethern mit unterschiedlichen Funktionalisierungsgraden.

Ist in der vorstehend aufgeführte Formel III von m-wertigen Resten R⁷ die Rede, so kann es sich dabei um beliebige ein- bis zwölfwertige Reste handeln.

Bei den m-wertigen Resten R⁷ kann es sich beispielsweise um aliphatische, cycloaliphatische, aromatische, araliphatische oder hetero-cyclische Reste handeln, bei denen ein bis zwölf Bindungen für die Verbindung mit dem Rest Y bzw. R⁸ zur Verfügung stehen. Beispiele für ein- oder zweiwertige solcher Reste finden sich in den vorstehenden Abschnitten bei der Beschreibung der Alkyl, Cycloalkyl, Aryl-, Aralkyl-, Heterocyclyl-, Alkylen-, Alkylenether-, Cycloalkylen-, Arylen-, Aralkylen- und Heterocyclenreste. Höherwertige Reste R₇ mit z.B. m = 3 oder 4 besitzen entsprechende Strukturen mit jedoch weiteren freien Valenzen für die Bindung an Y bzw. R⁸.

Beispiele für höherwertige Reste R⁷ mit z.B. m = 2 bis 12 sind Polysiloxane, in denen 2 bis 12 Siliziumatome mit Allyl- oder Methallylgruppen oder mit Allylether- oder Methallylethergruppen funktionalisiert sind; oder Kohlehydrate, in denen 2 bis 12 Hydroxygruppen mit Allyl- oder Methallylgruppen verethert sind, oder Polyvinylalkohole, in denen 2 bis 12 Hydroxygruppen mit Allyl- oder Methallylgruppen funktionalisiert sind.

Beispiele für (Meth)allylverbindungen mit mehr als vier Allylgruppen sind Polyallylverbindungen in der Form von ungesättigten Polyestern mit allylischen und/oder methallylischen Seitenketten, insbesondere styrolfreie Gemische dieser Polyester.

Beispiele für (Meth)allylverbindungen mit sechs Allylgruppen und/oder Methallylgruppen sind Mannit-hexaallylether, Sorbit-hexyallylether oder Inosithexaallylether sowie die entsprechenden Methallylderivate.

Beispiel für (Meth)allylverbindungen mit mehr als sechs Allylgruppen und/oder Methallylgruppen sind Polysaccharide mit mehr als sechs Allylgruppen und/oder Methallylgruppen.

Bei den m-wertigen Resten R⁷ kann es sich auch um weitere Reste handeln, bei denen ein bis zwölf Bindungen für die Verbindung mit dem Rest Y bzw. R⁸ zur Verfügung stehen. Beispiele für weitere Reste sind Harnstoff-, Cyanamid-, Phosphonat-, Carbonat-, Cyanurat-, Isocyanurat-, Pyrocarbonat-, Monoalkylsilan-, Dialkylsilan-, Trialkylsilan-, Monoarylsilan-, Diarylsilan-, Triarylsilan-, Tetraalkyldisiloxan, Tetraaryldisiloxan-, Tetrakis(trialkylsiloxy)disiloxan-, Thioharnstoff-, Sulfid-, Sulfon-, Boran-, Phosphat-, Phosphit- und Thioharnstoffreste. Diese weisen vorzugsweise ein bis zwei Allylgruppen oder Methallylgruppen auf.

Die m-wertigen organische Reste R⁷ können gegebenenfalls substituiert sein, beispielsweise mit ein oder mehreren Alkyl-, Alkoxy-, Amino- oder Hydroxyresten oder mit ein oder mehreren Halogenatomen, beispielsweise Chloratomen, oder mit Kombinationen von zwei oder mehreren dieser Substituenten.

Bei mehrwertigen Resten R⁷ müssen nicht sämtliche Funktionalitäten mit Resten R⁸ verbunden sein. Es ist durchaus möglich, dass nur einige dieser funktionellen Gruppen mit solchen Resten verbunden sind und dass die übrigen funktionellen Gruppen nicht-derivatisiert sind oder mit anderen gesättigten und/oder von Maleinsäure- bzw. Fumarsäure(derivaten) abgeleiteten Resten verbunden sind. Es lassen sich auch Gemische von verschiedenen Inhaltsstoffen b) einsetzen.

Neben den zuvor beschriebenen Substituenten kann R⁷ auch Säurefunktionen, wie z.B. Phosphorsäuregruppen, Phosphonsäuregruppen, Sulfonsäuregruppen und/oder Carbonsäuregruppen sowie deren Anhydride enthalten. Dentalmassen mit solchen freie Säuregruppen enthaltenden Verbindungen eignen sich insbesondere zur Verwendung als selbstätzende Dentalzemente.

Beispiele für besonders bevorzugte erfindungsgemäß eingesetzte Reaktiv-Pastenbildner b) mit Säurefunktionen sind die folgenden Verbindungen (R = H oder Methyl):

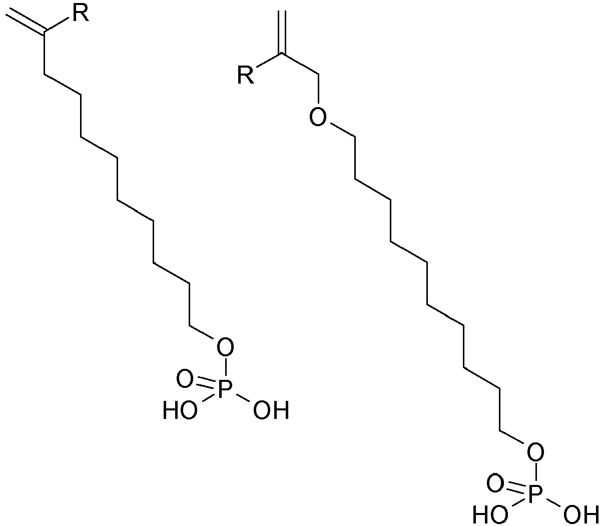

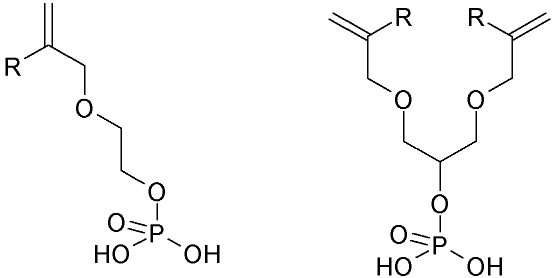

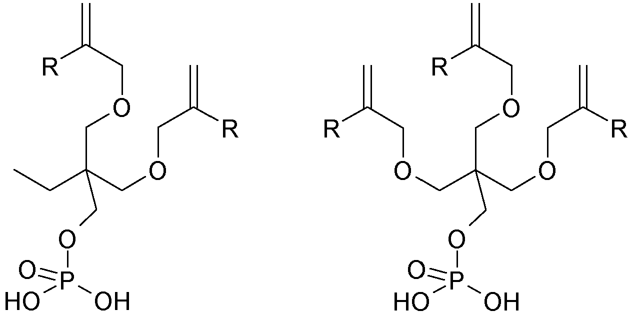

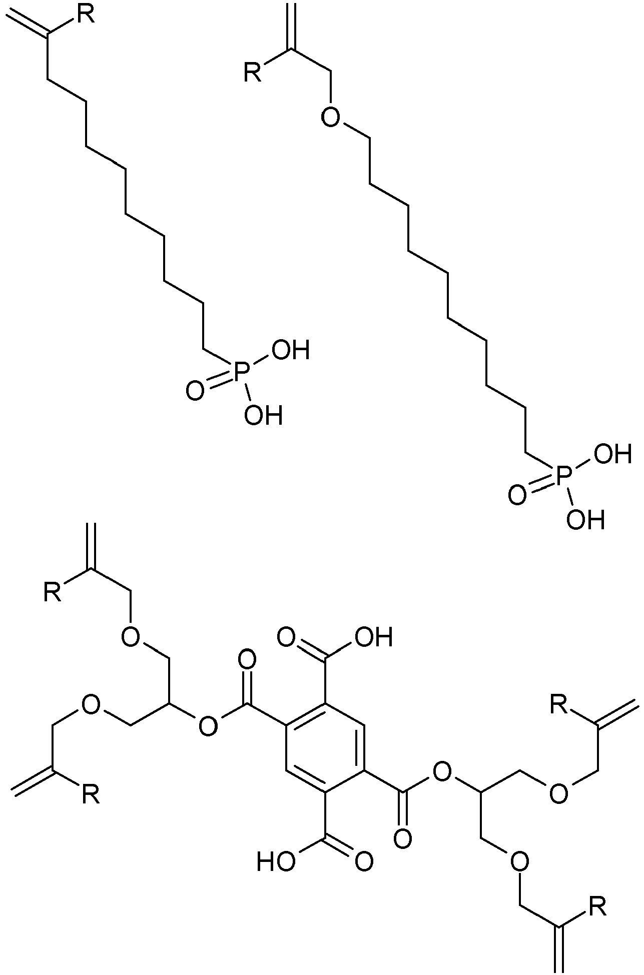

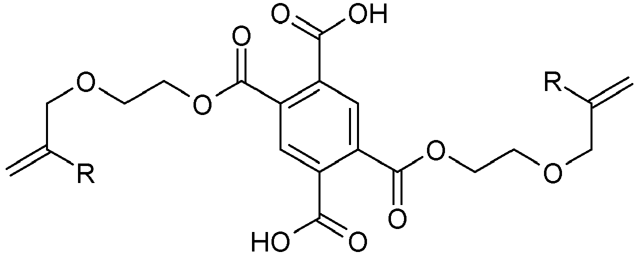

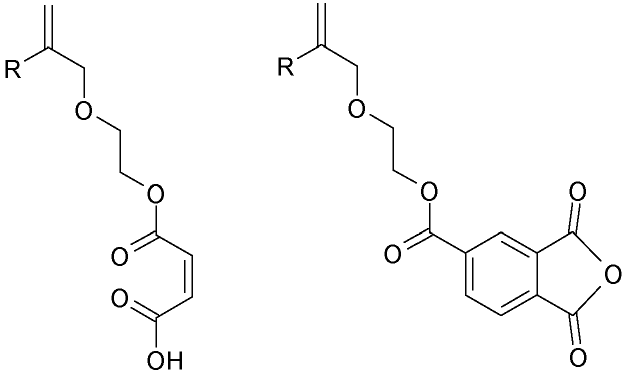

Insbesondere auch in diesem Zusammenhang kann es sinnvoll sein, wenn die bevorzugt erfindungsgemäß eingesetzten Reaktiv-Pastenbildner b) neben den Säurefunktionen auch freie Hydroxylgruppen am Substituenten R⁷ aufweisen und/oder wenn Gemische von säurefunktionalisierten Reaktiv-Pastenbildnern b) mit hydroxyfunktionalisierten Reaktiv-Pastenbildnern b) eingesetzt werden.

Beispiele für besonders bevorzugte erfindungsgemäß eingesetzte hydroxylfunktionalisierte Reaktiv-Pastenbildner b) sind die folgenden Verbindungen (R = H oder Methyl):

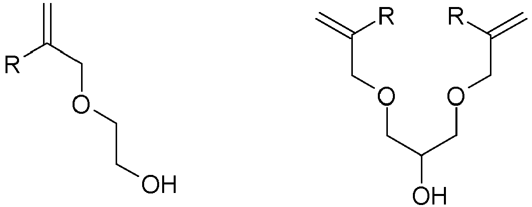

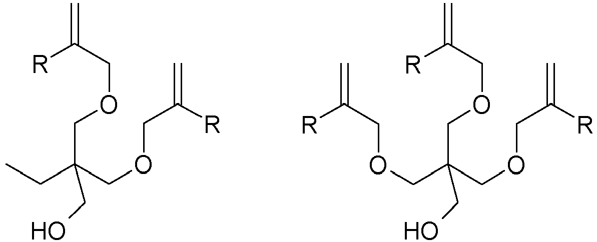

Ist in der vorstehend aufgeführte Formel III von Y als zweiwertigem Rest die Rede, so kann es sich dabei um beliebige zweiwertige organische oder anorganische Reste handeln. Bei Y kann es sich um eine beliebige Brückengruppe handeln oder um eine kovalente Bindung, welche die Reste R⁷ und R⁸ miteinander verbinden.

Beispiele für zweiwertige Reste Y sind die weiter oben aufgezählten, gegebenenfalls substituierten, zweiwertigen Reste, also Alkylen, Alkylenether, Cycloalkylen, Arylen, Aralkylen und Heterocyclen. Weitere Beispiele für zweiwertige Reste Y sind Carbonsäure-, Carbonsäureester-, Carbonsäureamid- oder Amidgruppen sowie Sauerstoff- oder Schwefelatome.

Beispiele für besonders bevorzugte erfindungsgemäß eingesetzte Reaktiv-Pastenbildner b) sind Verbindungen der Formel IIIa

CH₂=CR⁹-CH₂-Spa-Z-Spa-CH₂-CR⁹=CH₂ (IIIa),

worin R⁹ Wasserstoff oder Methyl ist,
Spa eine kovalente Bindung zwischen 2 C-Atomen bedeutet oder eine zweiwertige Brückengruppe ist, vorzugsweise ein Sauerstoffatom, eine Aminogruppe, Alkylen, Alkylenglykolether, Cycloalkylen, Arylen, Aralkylen oder Heterocyclylen, und
Z ein zweiwertiger polycyclischer Rest oder ein zweiwertiger ein- oder zweikerniger aromatischer Rest ist, vorzugsweise ein Rest der nachstehenden Formeln IIIb oder IIIc 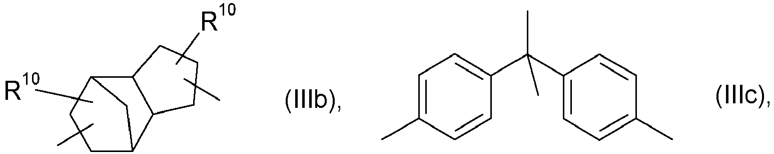
worin R¹⁰ Wasserstoff, Hydroxyl, Alkoxy oder Amino, insbesondere NH₂, bedeutet.

Beispiele für besonders bevorzugte erfindungsgemäß eingesetzte Reaktiv-Pastenbildner b) sind Verbindungen der Formel IIId und der Formel IIIe: 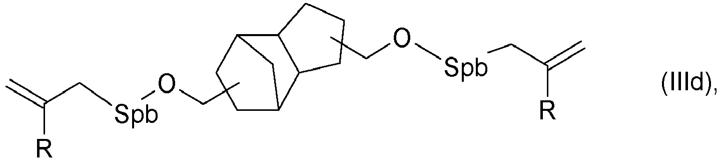 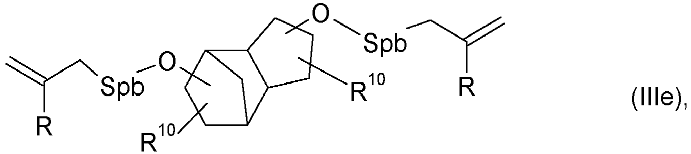
worin R Wasserstoff oder Methyl bedeutet,
Spb eine kovalente Bindung zwischen einem C-Atom und einem O-Atom bedeutet oder eine Brückengruppe darstellt, vorzugsweise Alkylen, Alkylenglykolether, Cycloalkylen, Arylen, Aralkylen oder Heterocyclylen, und
R¹⁰ Wasserstoff oder ein Hydroxy-, Alkoxy- oder Aminorest bedeutet.

Ganz besonders bevorzugte Beispiele für Verbindungen der Formel IIId sind Verbindungen der nachstehenden Formel 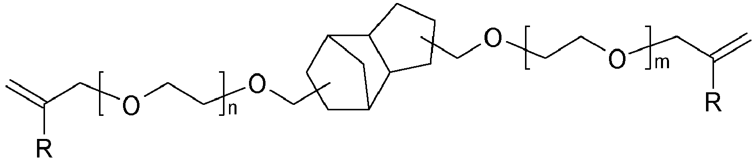
mit n = 0, 1, 2, ... 15 und m = 0, 1, 2, ... 15 sowie R = Wasserstoff oder Methyl.
Ganz besonders bevorzugte Beispiele für Verbindungen der Formel IIIe sind Verbindungen der nachstehenden Formel 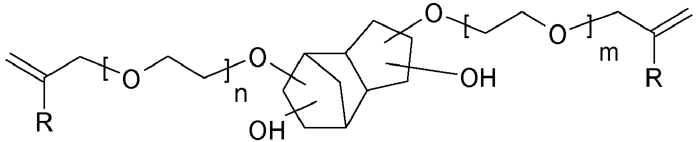
mit n = 0, 1, 2, ... 15 und m = 0, 1, 2, ... 15 sowie R = Wasserstoff oder Methyl, wobei die (Meth)allylethergruppen und die Hydroxylgruppen vorzugsweise jeweils an benachbarten Ringkohlenstoffatomen angebracht sind.

Alternativ zu den dargestellten Verbindungen mit Ethylenglykol-Einheiten [-O-CH₂-CH₂-]ₙ können auch vorzugsweise Verbindungen mit Propylenglykol-Einheiten [-O-CH₂-CH(CH₃)-]ₙ oder mit Butylenglykol-, wie Tetramethylenglykoleinheiten, [-O-CH₂-CH₂-CH₂-CH₂-]ₙ oder mit Cyclopentyleneinheiten [-O-C₅H₈-]ₙ oder mit Cyclohexyleneinheiten [-O-C₆H₁₀-]ₙ oder mit Phenyleneinheiten [-O-C₆H₄-]ₙ oder mit Naphthyleneinheiten [-O-C₁₀H₆-]ₙ oder mit Benzyleneinheiten [-O-CH₂-C₆H₄-]ₙ eingesetzt werden, wobei n eine ganze Zahl von 1 bis 15 bedeuten kann. Entsprechendes gilt für Ethylenglykoleinheiten [-O-CH₂-CH₂-]ₘ.

Weitere Beispiele für besonders bevorzugte erfindungsgemäß eingesetzte Reaktiv-Pastenbildner b) sind die Verbindungen der nachstehenden Formeln: 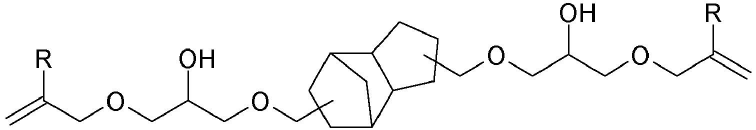 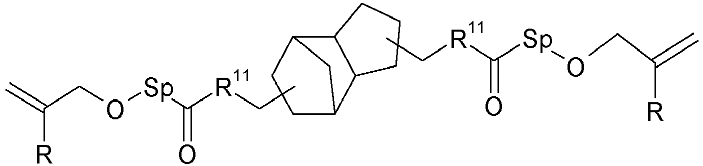 mit
Sp = Brückengruppe, vorzugsweise Alkylen oder Alkylenglykolether,
R= Wasserstoff oder Methyl, und
R¹¹= Sauerstoffatom oder-NH-Gruppe. 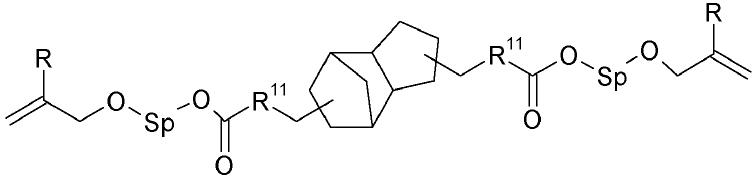 mit
   Sp = Brückengruppe, vorzugsweise Alkylen oder Alkylenglykolether,
   R¹¹= Sauerstoffatom oder-NH-Gruppe, und
   R= Wasserstoff oder Methyl. 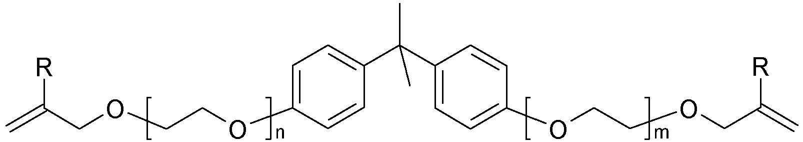
   mit n = 0, 1, 2, ... 15 und m = 0, 1, 2, ... 15 sowie R= Wasserstoff oder Methyl. 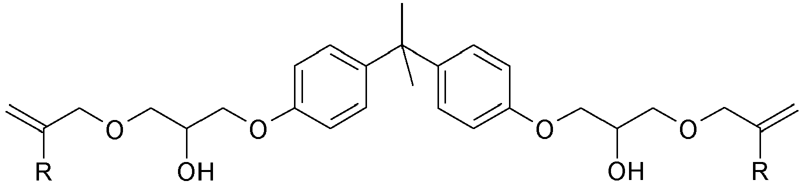
   mit R= Wasserstoff oder Methyl. 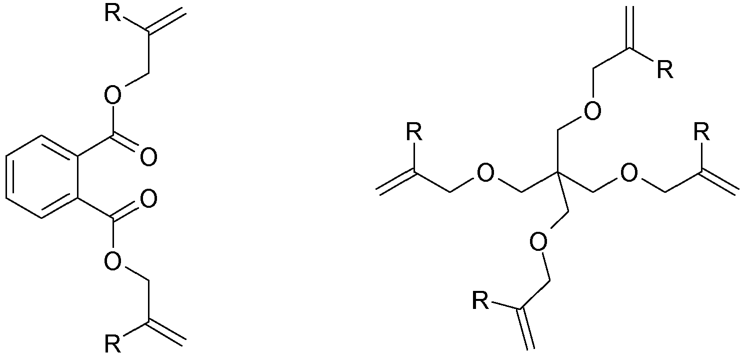
   mit R = H, Alkyl, insbesondere Methyl mit R = H, Alkyl, insbesondere Methyl. Dabei kann der Pentaerytrithether vorzugsweise als technisches Gemisch des vierfach-funktionalisierten Ethers mit niedriger funktionalisierten Ethern eingesetzt werden, beispielsweise als technisches Gemisch enthalten die vier-, drei- und zweifach-funktionalisierten Pentaerytrithether.

Weitere Beispiele für besonders bevorzugte erfindungsgemäß eingesetzte Reaktiv-Pastenbildner b) sind die folgenden Verbindungen:
Diallylmalat, Diallylharnstoff, N,N-Diallylacrylamid, Diallylsebacat, Diallylterephthalat, Diallylmalonat, Diallyloxalat, Diallylcyanamid, Diallylglutarat, Diallyldiglykolat, Diallylfumarat, Diallylhomophthalat, Diallylether, Diallylallylphosphonat, Diallyl-suberat, Diallylsuccinat, N,N-Diallyl(meth)-acrylamid, Diallylisophthalat, Diallylmaleat, Diallylazelat, Diallylcarbonat, Trimethylolpropandiallylether, Glycerin-α,α'-diallylether, Isocyanursäurediallyl-n-propylester, Isocyanursäurediallylester, Pyrocarbonsäurediallylester, Diethyldiallylmalonat, Diallylphthalat, Diallyldiphenylsilan, 1,3-Diallyltetra-methyldisiloxan, Diallyldimethylsilan, 1,3-Diallytetrakis(trimethylsiloxy)disiloxan, Diallylmaleat, N,N"-Diallylthioharnstoff, Diallylsulfid, Diethyl-2,2-diallylmalonat, Diallylsulfon, Diallyladipat (C₁₂H₁₈O₄), 1,2-Diallyl-cyclohexan-1,4-dicarboxylat (C₁₄H₂₀O₄; cis-/trans-Mischung), Triallylboran, Triallylphosphat, Triallytrimesat, Triallylaconitat, Triallylcitrat, Triallylisocyanurat, Pentaerythritoltriallylether, Triallylcyanurat, Triallylphosphit, Triallylthioharnstoff, Triallyltrimellitat, Isocyanursäuretriallylester, Tetraallylpyromellitat, 1,1,3,3-Tetraallyloxypropan, Tetraallylpentaerythritol und Tetraallyloxyethan bzw. die analogen Methallyl-verbindungen.

Unter Barbitursäurederivaten c) sind im Rahmen der vorliegenden Beschreibung Barbitursäure, Thiobarbitursäure und insbesondere substituierte Barbitursäuren und substituierte Thiobarbitursäuren zu verstehen. Salze von Barbitursäure und deren Derivaten sowie von Thiobarbitursäure und deren Derivaten sind ausgeschlossen.

Auch Salze von Malonylsulfamiden sind ausgeschlossen.

Bei Inhaltsstoff c) kann es sich um beliebige Barbitursäurederivate und/oder Malonylsulfamide handeln. Neben Barbitursäure können auch deren Derivate, vorzugsweise die in 1-, 3- und/oder 5-Position substituierten Verbindungen oder die entsprechenden Thiobarbiturate eingesetzt werden. Besonders bevorzugt handelt es sich dabei um Barbitursäurederivate der Formel IV oder V oder um Malonylsulfamide der Formel VI 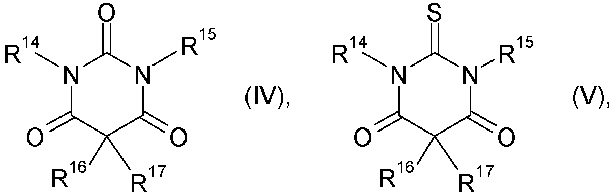 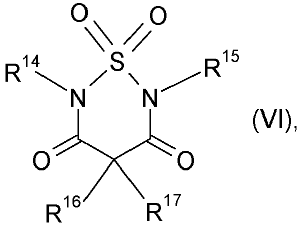
worin R¹⁶ und R¹⁷ unabhängig voneinander Wasserstoff, Alkyl, Alkenyl, Cycloalkyl, Aryl, Aralkyl und Heterocyclyl bedeuten, mit der Maßgabe, dass mindestens einer der Reste R¹⁶ oder R¹⁷ Wasserstoff bedeutet, und
R¹⁴ und R¹⁵ unabhängig voneinander Wasserstoff, Alkyl, Alkenyl, Cycloalkyl, Aryl, Aralkyl und Heterocyclyl bedeuten.

Ganz besonders bevorzugt werden Barbitursäurederivate der Formel IV eingesetzt, worin R¹⁴ und/oder R¹⁶ oder R¹⁷ unabhängig voneinander Alkyl, Cycloalkyl, Aryl oder Aralkyl bedeuten, insbesondere Ethyl, Butyl, Phenyl oder Benzyl.

Beispiele für besonders bevorzugte erfindungsgemäß eingesetzte Polymerisationsinitiatoren c) sind die in der DE 1 495 520 beschriebenen Barbitursäuren und Barbitursäurederivate sowie die in der EP 0 059 451 B1 beschriebenen Malonylsulfamide.

Bevorzugte Malonylsulfamide sind 2,6-Dimethyl-4-isobutylmalonylsulfamid, 2,6-Diisobutyl-4-propylmalonylsulfamid, 2,6-Dibutyl-4-propylmalonylsulfamid, 2,6-Dimethyl-4-ethyl-malonylsulfamid sowie 2,6-Dioctyl-4-isobutylmalonylsulfamid.

Beispiele für besonders bevorzugt eingesetzte Barbitursäurederivate sind 1-Benzyl-5-phenylbarbitursäure, 1-Cyclohexyl-5-ethylbarbitursäure, 1,3-Dimethyl-5-cyclopentylbarbitursäure, 1,3-Dimethyl-5-cyclohexylbarbitursäure, 1,3-Dimethyl-5-ethylbarbitursäure, 1,3-Dimethyl-5-isobutylbarbitursäure, 1,3-Dimethyl-5-phenylbarbitursäure, 1,3-Dimethyl-5-n-butylbarbitursäure, 1,5-Dimethylbarbitursäure, 5-n-Butylbarbitursäure, 5-Cyclohexylbarbitursäure, 5-Ethylbarbitursäure, 5-Isobutylbarbitursäure, 5-Isoproypylbarbitursäure, 5-Phenylbarbitursäure und 1,3,5-Trimethylbarbitursäure. Diese Barbitursäure-Derivate können alleine oder als eine Mischung von zweien oder mehreren davon verwendet werden.

Ist in den vorstehend aufgeführten Formeln I bis VI von Alkylresten die Rede, so handelt es sich dabei um gesättigte verzweigte oder unverzweigte aliphatische Kohlenwasserstoffreste. Deren Kettenlänge beträgt in der Regel bis zu 10 C-Atomen. Bevorzugt werden C₁ bis C₈-Alkylreste. Beispiele dafür sind Methyl, Ethyl, n-Propyl, Isopropyl, n-Butyl, sek.-Butyl, tert.-Butyl, n-Pentyl, n-Hexyl oder 2-Ethylhexyl. Alkylreste können gegebenenfalls substituiert sein, beispielsweise mit ein oder mehreren Alkoxy-, Amino- oder Hydroxyresten oder mit sauren Resten, wie z.B. mit Phosphorsäuregruppen, Phosphonsäuregruppen, Sulfonsäuregruppen und/oder Carbonsäuregruppen sowie deren Anhydriden oder mit ein oder mehreren Halogenatomen, beispielsweise Chloratomen, oder mit Kombinationen von zwei oder mehreren dieser Substituenten.

Ist in den vorstehend aufgeführten Formeln IV bis VI von Alkenylresten die Rede, so handelt es sich dabei um einfach ungesättigte verzweigte oder unverzweigte aliphatische Kohlenwasserstoffreste. Deren Kettenlänge beträgt in der Regel bis zu 10 C-Atomen. Bevorzugt werden C₂ bis C₈-Alkenylreste. Beispiele dafür sind Allyl, Methallyl oder Vinyl. Alkenylreste können gegebenenfalls substituiert sein, beispielsweise mit ein oder mehreren Alkoxy-, Amino- oder Hydroxyresten oder mit sauren Resten, wie z.B. mit Phosphorsäuregruppen, Phosphonsäuregruppen, Sulfonsäuregruppen und/oder Carbonsäuregruppen sowie deren Anhydriden, oder mit ein oder mehreren Halogenatomen, beispielsweise Chloratomen, oder mit Kombinationen von zwei oder mehreren dieser Substituenten.

Ist in den vorstehend aufgeführten Formeln I bis VI von Cycloalkylresten die Rede, so handelt es sich dabei um gesättigte cycloaliphatische Kohlenwasserstoffreste. Diese weisen in der Regel fünf bis acht Ringkohlenstoffatome auf. Bevorzugt werden Cyclopentyl- oder insbesondere Cyclohexylreste. Diese Reste können einen oder mehrere Cycloalkylringe aufweisen. Mehrere Cycloalkylringe können miteinander durch kovalente Bindungen oder Brückengruppen verknüpft sein oder bi- oder polycyclische Ringsysteme bilden. Cycloalkylreste können gegebenenfalls substituiert sein, beispielsweise mit ein oder mehreren Alkyl-, Alkoxy-, Amino- oder Hydroxyresten oder mit sauren Resten, wie z.B. mit Phosphorsäuregruppen, Phosphonsäuregruppen, Sulfonsäuregruppen und/oder Carbonsäuregruppen sowie deren Anhydriden, oder mit ein oder mehreren Halogenatomen, beispielsweise Chloratomen, oder mit Kombinationen von zwei oder mehreren dieser Substituenten.

Ist in den vorstehend aufgeführten Formeln I bis VI von Arylresten die Rede, so handelt es sich dabei um aromatische Kohlenwasserstoffreste. Diese weisen in der Regel sechs bis zwölf Ringkohlenstoffatome auf. Bevorzugt werden Naphthyl- oder insbesondere Phenylreste. Mehrere Arylringe können miteinander durch kovalente Bindungen oder Brückengruppen verknüpft sein oder bi- oder polycyclische aromatische Ringsysteme bilden. Arylreste können gegebenenfalls substituiert sein, beispielsweise mit ein oder mehreren Alkyl-, Alkoxy-, Amino- oder Hydroxyresten oder mit sauren Resten, wie z.B. mit Phosphorsäuregruppen, Phosphonsäuregruppen, Sulfonsäuregruppen und/oder Carbonsäuregruppen sowie deren Anhydriden, oder mit ein oder mehreren Halogen-atomen, beispielsweise Chloratomen, oder mit Kombinationen von zwei oder mehreren dieser Substituenten.

Ist in den vorstehend aufgeführten Formeln I bis VI von Aralkylresten die Rede, so handelt es sich dabei um aromatische Kohlenwasserstoffreste verbunden mit einem Alkylenrest. Diese Reste weisen in der Regel sechs bis zwölf Ringkohlenstoffatome und ein bis drei Kohlenstoffatome im Alkylenrest auf. Ein bevorzugtes Beispiel für Aralkylreste ist Benzyl. Aralkylreste können gegebenenfalls substituiert sein, beispielsweise mit ein oder mehreren Alkyl-, Alkoxy-, Amino- oder Hydroxyresten oder mit sauren Resten, wie z.B. mit Phosphorsäuregruppen, Phosphonsäuregruppen, Sulfonsäuregruppen und/oder Carbonsäuregruppen sowie deren Anhydriden, oder mit ein oder mehreren Halogenatomen, beispielsweise Chloratomen, oder mit Kombinationen von zwei oder mehreren dieser Substituenten.

Ist in den vorstehend aufgeführten Formeln I bis VI von Alkylenresten die Rede, so handelt es sich dabei um gesättigte verzweigte oder unverzweigte zweiwertige aliphatische Kohlenwasserstoffreste. Deren Kettenlänge beträgt in der Regel bis zu 8 C-Atomen. Bevorzugt werden C₁ bis C₆-Alkylreste. Beispiele dafür sind Methylen, Ethylen, n-Propylen, Isopropylen, n-Butylen, sek.-Butylen, tert.-Butylen, n-Pentylen und n-Hexylen. Alkylenreste können gegebenenfalls substituiert sein, beispielsweise mit ein oder mehreren Alkoxy-, Amino- oder Hydroxyresten oder mit sauren Resten, wie z.B. mit Phosphorsäuregruppen, Phosphonsäuregruppen, Sulfonsäuregruppen und/oder Carbonsäuregruppen sowie deren Anhydriden, oder mit ein oder mehreren Halogenatomen, beispielsweise Chloratomen, oder mit Kombinationen von zwei oder mehreren dieser Substituenten.

Ist in den vorstehend aufgeführten Formeln I bis VI von Alkylenglykoletherresten die Rede, so handelt es sich dabei Reste, die sich von gesättigten verzweigten oder unverzweigten aliphatischen Alkylenglykolen ableiten. Diese Reste können eine oder mehrere wiederkehrende Struktureinheiten aufweisen. Die Alkyleneinheiten weisen in der Regel zwei bis vier Kohlenstoffatome auf. Beispiele für Reste dieses Typs sind -[C₂H₄-O]_{q}- C₂H₄-, -[C₃H₆-O]_{q}- C₃H₆- und -[C₄H₈-O]_{q}-C₄H₈- mit q = 1-100, vorzugsweise 1-30, insbesondere 1-15. Alkylenglykoletherreste können gegebenenfalls substituiert sein, beispielsweise mit ein oder mehreren Alkoxy-, Amino- oder Hydroxyresten oder mit ein oder mehreren Halogenatomen, beispielsweise Chloratomen, oder mit Kombinationen von zwei oder mehreren dieser Substituenten.

Bei Inhaltsstoff d) kann es sich um beliebige mindestens eine Acrylsäureester- und/oder Methacrylsäureesterreste umfassende organische Verbindungen handeln. Neben monomeren Verbindungen eignen sich auch oligomere und polymere (Meth)acrylate, vorausgesetzt diese weisen noch polymerisationsfähige (Meth)acrylestergruppen auf. Besonders bevorzugt handelt es sich dabei um Verbindungen der Formel VII 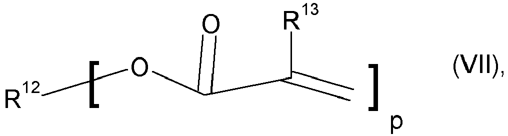
worin p eine ganze Zahl von 1 bis 12, vorzugsweise 1 bis 4 ist,
R¹² ein p-wertiger organischer Rest ist, der gegebenenfalls einen oder mehrere Substituenten aufweist, und
R¹³ Wasserstoff oder Methyl bedeutet.

Ist in der vorstehend aufgeführte Formel VII von einem p-wertigen organischen Rest R¹² die Rede, so kann es sich dabei um beliebige ein- bis zwölfwertige organische Reste handeln.

Bei den p-wertigen organischen Resten R¹² kann es sich dabei um aliphatische, cycloaliphatische, aromatische, araliphatische oder heterozyclische Reste handeln, bei denen ein bis zwölf Bindungen für die Verbindung mit dem (Meth)acrylatrest zur Verfügung stehen. Beispiele für ein- oder zweiwertige solcher Reste R¹² finden sich in den vorstehenden Abschnitten bei der Beschreibung der Alkyl, Cycloalkyl, Aryl-, Aralkyl-, Heterocyclyl-, Alkylen-, Alkylenether-, Cycloalkylen-, Arylen-, Aralkylen- und Heterocyclcylenreste. Höherwertige Reste mit p = 3 bis 12 besitzen entsprechende Strukturen mit jedoch weiteren freien Valenzen für die Bindung an die (Meth)acrylatgruppe(n). Alternativ kann es sich bei den p-wertigen organischen Resten auch um Reste eines beliebigen Polymers handeln, das z.B. mit 1-6 (Meth)acrylatgruppen funktionalisiert ist.

Beispiele für höherwertige Reste R¹² mit z.B. m = 2 bis 12 sind Polysiloxane, in denen 2 bis 12 Siliziumatome mit oder mit Acrylat- oder Methacrylatgruppen funktionalisiert sind; oder Kohlehydrate, in denen 2 bis 12 Hydroxygruppen mit Acryat- oder Methacrylatgruppen verestert sind, oder Polyvinylalkohole, in denen 2 bis 12 Hydroxygruppen mit Acrylat- oder Methacrylatgruppen funktionalisiert sind.

Bei mehrwertigen organischen Resten R¹² müssen nicht sämtliche Funktionalitäten mit (Meth)acrylatresten verbunden sein. Es ist durchaus möglich, dass nur einige dieser funktionellen Gruppen mit solchen Resten verbunden sind und dass die übrigen funktionellen Gruppen nicht-derivatisiert sind oder mit anderen gesättigten und/oder ungesättigten Resten verbunden sind. Es lassen sich auch Gemische von verschiedenen Inhaltsstoffen d) einsetzen.

Die p-wertigen organischen Reste R¹² können gegebenenfalls substituiert sein, beispielsweise mit ein oder mehreren Alkyl-, Alkoxy-, Amino- oder Hydroxyresten oder mit sauren Resten, wie z.B. mit Phosphorsäuregruppen, Phosphonsäuregruppen, Sulfonsäuregruppen und/oder Carbonsäuregruppen sowie deren Anhydriden, oder mit ein oder mehreren Halogenatomen, beispielsweise Chloratomen, oder mit Kombinationen von zwei oder mehreren dieser Substituenten.

Beispiele für bevorzugt eingesetzte (Meth)acrylatverbindungen d) sind mindestens bifunktionelle Acrylsäure- und/oder (Meth)acrylsäureester. Dabei kann es sich um monomere oder um polymere Acrylate und Methacrylate handel. Vorteilhaft verwendet werden können beispielsweise die langkettigen Monomere der US 3,066,112 auf der Basis von Bisphenol-A und Glycidyl(meth)acrylat oder deren durch Addition von Isocyanaten entstandenen Derivate. Besonders geeignet sind auch Verbindungen des Typs Bisphenol-A-diethyloxy(meth)acrylat und Bisphenol-A-dipropyloxy(meth)acrylat. Weiterhin bevorzugte Verwendung finden können oligo-ethoxylierte und oligo-propoxylierte Bisphenol-A-diacryl- und -di(meth)acrylsäureester.

Gut geeignet sind weiter Acrylsäure- und Methacrylsäurediester oder höhere Ester mindestens bifunktioneller aliphatischer Alkohole, beispielsweise Triethylenglykol-di(meth)acrylat, Ethylenglykol-di(meth)acrylat, Hexandiol-di(meth)acrylat oder Trimethylolpropan-tri(meth)acrylat.
Besonders geeignet sind auch die in der DE 28 16 823 C2 genannten Diacryl- und Dimethacrylsäureester des Bis(hydroxymethyl)-tricyclo[5.2.1.0^{2,6}]-decans und die Diacryl- und Di(meth)acrylsäureester der mit 1 bis 3 Ethylenoxid- und/oder Propylenoxideinheiten verlängerten Verbindungen des Bis(hydroxymethyl)-tricyclo[5.2.1.0^{2,6}]-decans.

Gut geeignete Monomere d) sind zudem die in der EP 0 235 826 A1 beschriebenen (Meth)acrylsäureester, z.B. Triglykolsäure-bis[3(4)-(meth)acryloxymethyl-8(9)-tricyclo[5.2.1.0^{2,6}]-decylmethylester].

Selbstverständlich können auch Gemische aus Monomeren und/oder hieraus hergestellten ungesättigten Polymeren verwendet werden.

In einer weiteren bevorzugten Ausführungsform können zusätzlich zu mindestens bifunktionellen Acrylsäure- und Methacrylsäureestern bis 70 %, bezogen auf Inhaltsstoff d), vorzugsweise bis 50 % monofunktionelle (Meth)acrylsäureester, wie Methyl(meth)acrylat, eingesetzt werden.

Weitere bevorzugte Beispiele von (Meth)acrylaten d) schließen ein:
Methyl(meth)acrylat, Ethyl(meth)acrylat, Isopropyl(meth)acrylat, n-Butyl-(meth)acrylat, Isobutyl(meth)acrylat, Hydroxypropyl(meth)acrylat, Tetrahydrofurfuryl(meth)acrylat, Glycidyl(meth)acrylat, 2-Hydroxyethyl(meth)acrylat, 2-Hydroxypropyl(meth)acrylat, 3-Hydroxypropyl(meth)acrylat, 2-Methoxyethyl-(meth)acrylat, 2-Ethoxyethyl(meth)acrylat, 2-Methoxyethyl(meth)acrylat, 2-Ethylhexyl(meth)acrylat, Benzyl(meth)acrylat, 2-Hydroxy-1,3-di(meth)acryloxy-propan;
Neopentylglykoldi(meth)acrylat, 1,3-Butandioldi(meth)acrylat, 1,4-Butandiol-di(meth)acrylat, 1,6-Hexandioldi(meth)acrylat, 1,8-Oktandioldi(meth)acrylat, 1,10-Decandioldi(meth)acrylat, 1,12-Dodecandioldi(meth)acrylat, 1,14-Tetra-decandioldi(meth)acrylat, 1,16-Hexadecandioldi(meth)acrylat, Trimethylol-propantri(meth)acrylat, Trimethylolethantri(meth)acrylat, Pentaerythritol-tri(meth)acrylat, Trimethylolmethantri(meth)acrylat, Pentaerythritoltetra-(meth)acrylat, Bisphenol-A-diglycidyl(meth)acrylat, Mono- oder Polyethylen-glykoldi(meth)acrylat, z.B. Ethylenglykoldi(meth)acrylat, Diethylenglykoldi-(meth)acrylat und Triethylenglykoldi(meth)acrylat, Mono- oder Polypropylen-glykoldi(meth)acrylat, und Mono- oder Polybutylenglykoldi(meth)acrylat, z.B. Polytetrahydrofurandi(meth)acrylat, wobei bei den Polyethylenglykol-, Polypropylenglykol- und Polybutyenglykol- bzw. Polytetramethylenglykolderivaten sowohl solche mit verzweigter als auch mit linearer Struktur umfasst sind.

Zudem schließen Beispiele der (Meth)acrylate mit Urethan-Bindung(en) Di-2-(meth)acryloxyethyl-2,2',4-trimethylhexamethylendicarbamat, Di-2-(meth)acryl-oxyethyl-2,4,4'-trimethylhexamethylendicarbamat und 1,3,5-tris[1,3-bis{(meth)-acryloyloxy}-2-propoxycarbonylaminohexan]-1,3,5-(1H,3H,5H)triazin-2,4,6-trion mit ein. Daneben wird exemplarisch ein (Meth)acrylat eines Urethanoligomers genannt, das 2,2'-Di(4-hydroxycyclohexyl)propan, 2-Oxepanon, Hexamethylendiisocyanat and 2-Hydroxyethyl(meth)acrylat aufweist, und ein (Meth)acrylat eines Urethanoligomers, das 1,3-Butandiol, Hexamethylendiisocyanat und 2-Hydroxyethyl(meth)acrylat aufweist. Diese (Meth)acrylate können alleine oder als eine Mischung von zwei oder mehr verwendet werden.

Bevorzugt sind Inhaltsstoffe d), welche Tetrahydrofurfuryl(meth)acrylat, Glycidyl-(meth)acrylat, 2-Hydroxyethyl(meth)acrylat, 2-Hydroxypropl(meth)acrylat, 3-Hydroxypropyl(meth)acrylat und 2-Hydroxy-1,3-di(meth)acrylat enthalten. Weil sie Barbitursäure-Derivate bzw. Malonylsulfamide und organische Halogenide gut lösen, sind die obengenannten Verbindungen bevorzugt in Inhaltsstoff d) enthalten.

Das erfindungsgemäße Dentalmaterial enthält ferner in Komponente B Metallverbindungen als Inhaltsstoff e).

Bei Inhaltsstoff e) kann es sich um Metallverbindungen wie unten angegeben handeln. Diese dienen als Katalysator für die Bildung freier Radikale. In der Regel werden Metallsalze und/oder Metallkomplexe eingesetzt.

Bei den Metallen des Inhaltsstoffes e) handelt es sich um Metalle der dritten und vierten Hauptgruppe sowie der ersten bis achten Nebengruppe des Periodensystems der Elemente einschließlich der Lanthaniden. Diese Metallverbindungen kommen vorzugsweise als Salze oder Komplexverbindungen des Kupfers, Eisens, Zinns, Chroms, Mangans, Kobalts, Zinks, Nickels, der Seltenen Erden und des Aluminiums zum Einsatz.

Die Metalle können in verschiedenen nicht-negativen Oxidationsstufen vorliegen, beispielsweise in der Oxidationsstufe +1 oder insbesondere +2. Metalle des Inhaltsstoffs e) können auch in unterschiedlichen Oxidationsstufen vorliegen bzw. es können Gemische unterschiedlichster Metallverbindungen eingesetzt werden.

Die Metallsalze können beliebige Anionen aufweisen. Beispiele für Anionen sind Halogenide, Pseudohalogenide, Sulfate, Sulfonate, Phosphate, Phosphonate und insbesondere Carboxylate, wie Anionen von aliphatischen oder aromatischen Carbonsäuren, z.B. (Meth)acrylsäureanionen.

Die Metallkomplexe können beliebige Liganden aufweisen. Beispiele für Liganden sind beliebige Lewis-Basen, die mit den Metallatomen koordinative Bindungen auszubilden vermögen. Bevorzugt werden Metallkomplexe mit Carbonylverbindungen und/oder mit Stickstoffatomen, besonders bevorzugt mit beta-Carbonylverbindungen, ganz besonders bevorzugt mit Acetylacetonat, als Liganden als Inhaltsstoff e) eingesetzt.

Beispiele für bevorzugte Metallkomplexe oder Metallsalze des Inhaltsstoffs e) sind Kupferacetylacetonat, Kupfer-4-cyclohexylbutyrat, Kupferacetat, Kupferoleat, Kupferethylhexanoat, Kupferacrylat, Kupfermethacrylat, Kupfernaphthenat, Manganacetylacetonat, Mangannaphthenat, Manganoctylat, Kobaltacetylacetonat, Kobaltnaphthenat, Zinkacetylacetonat, Zinknaphthenat, Nickelacetylacetonat, Nickelacetat, Chromacetylacetonat, Eisenacetylacetonat, Natriumnaphthenat und Seltenerdoctoat.

Metallverbindungen des Inhaltsstoffes e) können alleine oder in einer Mischung von zwei oder mehreren davon verwendet werden.

Bevorzugt eingesetzte Inhaltsstoffe e) sind Metallverbindungen, die in Komponente B gelöst vorliegen, insbesondere in Form gelöster organischer Verbindungen. Als Metall ist Kupfer besonders geeignet.

Das erfindungsgemäße Dentalmaterial enthält ferner in Komponente B Halogenid- oder Pseudohalogenidverbindungen als Inhaltsstoff f).

Bei Inhaltsstoff f) kann es sich um Halogenid- oder Pseudohalogenidverbindungen wie unten angegeben handeln, die in Form von löslichen Salzen in Komponente B eingesetzt werden. Unter löslichen Salzen sind solche Verbindungen zu verstehen, die sich zu mindestens 1 g pro Liter bei 25°c in der organischen Verbindung d) lösen.

Halogenidverbindungen sind im allgemeinen Fluoride, Chloride, Bromide oder lodide.

Pseudohalogenidverbindungen sind im allgemeinen Verbindungen mit den Anionen CN⁻, N₃⁻, OCN⁻, NCO⁻, CNO⁻, SCN⁻, NCS⁻ oder SeCN⁻.

Als Kationen kommen grundsätzlich beliebige Metallkationen, vorzugsweise jedoch die Metallkationen von Metallen der ersten und der zweiten Hauptgruppe des Periodensystems, insbesondere Kalium-, Natrium- und Lithiumkationen, in Frage; oder auch Ammonium- oder Phosphoniumkationen, insbesondere solche mit organischen Resten einschließlich der Hydrohalogenide von Aminen, insbesondere von tertiären Aminen.

Die Inhaltsstoffe f) sind Halogenide, insbesondere Chloride oder Bromide, oder Pseudohalogenide der Metalle der ersten oder der zweiten Hauptgruppe des Periodensystems der Elemente, insbesondere des Lithiums oder des Natriums, oder der quaternären Ammoniumionen, der quaternären Phosphoniumionen sowie Hydrohalogenide tertiärer Amine.

Beispiele für bevorzugte Inhaltsstoffe f) sind organische Halogenide, wie Benzyltributylammoniumchlorid, Benzyldimethylcetylammoniumchlorid, Benzyldimethylstearylammoniumchlorid, Benzyltriethylammoniumbromid, Benzyltrimethylammoniumchlorid, Cetylalkoniumchlorid, Cetylpyridinbromid, Cetylpyridinchlorid, Cetyltriethylammoniumbromid, Mono- bis Tetraallylalkylammoniumchloride, Mono- bis Tetraallylalkylammoniumbromide, insbesondere Diallyldimethylammoniumchlorid, Dodecyldimethylammoniumchlorid, Dilauryldimethylammoniumchlorid, Lauryldimethylammoniumchlorid, tetra-n-Butylammoniumbromid, tetra-n-Butyl-ammoniumchlorid, Tetradecyltrimethylammoniumbromid, Tetraethylammoniumbromid und Trioctylmethylammoniumchlorid, die entsprechenden Phosphoniumverbindungen oder Hydrohalogenide tertiärer Amine, insbesondere Hydrochloride tertiärer Amine.

Die Verbindungen des Inhaltsstoffes f) können alleine oder in einer Mischung von zwei oder mehreren davon eingesetzt werden.

In einer bevorzugten Ausführungsform enthält die erfindungsgemäße Mehrkomponentenzusammensetzung in mindestens einer Komponente mindestens einen Füllstoff g). Insbesondere enthält Komponente B mindestens einen Füllstoff g) oder Komponenten A und B enthalten jeweils mindestens einen Füllstoff g).

Bei den Füllstoffen (Inhaltsstoff g)) handelt es sich um anorganische oder um organische Materialien. Beispiele für anorganische Materialien sind Siliziumdioxid in seinen verschiedenen Modifikationen (wie z.B. Quarz, Cristobalit, Quarzgut), Feldspat, gemahlene Gläser, wie Bariumglas, Aluminaglas, Kaliumglas und Fluoraluminosilikatglas, sowie schwer lösliche Fluoride, wie CaF₂, YF₃, YbF₃, außerdem Kieselgele sowie Kieselsäure, insbesondere pyrogene Kieselsäure oder deren Granulate, oder synthetischer Zeolit, Kalziumphosphat, Aluminumsilikat, Kalziumsilikat, Magnesiumcarbonat, hydratisiertes Kalziumsilikat oder hydratisiertes Aluminiumsilikat (Kaolin). Diese Füllstoffe können einer Oberflächenbehandlung z.B. mit γ-(Meth)acryloxypropyl-trimethoxysilan, Vinyltrichlorsilan, Vinyltriethoxysilan, Vinyltrimethoxysilan, Vinyltriacetoxysilan, Vinyltri(methoxyethoxy)silan, Alkyltrichlorsilan, Alkyltriethoxysilan, Alkyltrimethoxysilan, Alkyltriacetoxysilan, Alkyltri(methoxyethoxy)-silan, insbesondere Methyltrichlorsilan, Methyltriethoxysilan, Methyltrimethoxysilan, Methyltriacetoxysilan und Methyltri(methoxyethoxy)silan unterzogen werden. Ferner können anwendbare organisch-anorganisch Kompositfüller durch Mischen der vorstehenden Füllstoffe mit einem polymerisierbaren Monomer oder Oligomer, Härten der Mischung und anschließendes Pulverisieren der gehärteten Mischung hergestellt werden.

Als Füllstoffe eignen sich auch bereits fertig pigmentierte Polymethyl-(meth)acrylatperlen oder andere pulverisierte organische Polymerisate. Zur Erhöhung der Flexibilität der Dentalmassen kann es auch vorteilhaft sein, lösliche organische Polymere einzusetzen. Geeignet sind z.B. Polyvinylacetat sowie Copolymere auf der Basis von Vinylchlorid/Vinylacetat, Vinylchlorid/Vinylisobutylether und Vinylacetat/Maleinsäuredibutylether.

Die Füllstoffe können alleine oder in einer Mischung von zwei oder mehreren davon verwendet werden. Die maximale mittlere Korngröße der anorganischen Füllstoffe beträgt vorzugsweise 15 µm, insbesondere 10 µm. Ganz besonders bevorzugt werden Füllstoffe mit einer mittleren Korngröße von < 5 µm eingesetzt.

Von den Füllstoffen sind Anteile an Kieselsäureanhydrid, Kieselsäure, hydratisiertes Kalziumsilikat und hydratisiertes Aluminiumsilikat (Kaolin) bevorzugt, weil diese selbst im Falle einer Lagerung über einen langen Zeitraum bewirken, dass das pastenartige polymerisierbare Dentalmaterial nicht geliert. Im übrigen können natürlich in Abhängigkeit von den Reaktiv-Pastenbildnern a) und/oder b) in den entsprechenden Pasten verschiedene Füllstoffe g) verwendet werden.

Zur Verwirklichung eines höheren Monomerumsatzes ist es ferner von Vorteil, wenn das polymerisierbare Dentalmaterial als Inhaltsstoff h) bis zu 5 Gew. %, bezogen auf die Gesamtmasse der Komponente A und/oder B, in welcher sich der Inhaltsstoff h) befindet, einer organischen Peroxidverbindung enthält. Inhaltsstoff h) befindet sich bevorzugt in Komponente A. Vorzugsweise handelt es sich bei Inhaltsstoff h) um einen Carbonsäure-Peroxyester, Kohlensäure-Peroxyester, Diacylperoxid, Perketal, Peracetal, Perether, Hydroperoxid, eine Persäure, oder um Kombinationen von zweien oder mehreren davon. Davon sind insbesondere Carbonsäure-Peroxyester, Kohlensäure-Peroxyester und Perketale bevorzugt. Ganz besonders bevorzugt verwendet man tert-Butylperoxy-3,5,5-trimethyl-hexanoat, tert-Butylperoxybenzoat, tert-Butylperoxy-2-ethylhexyl-carbonat oder Kombinationen von zwei oder mehreren davon.

Enthält das erfindungsgemäße Dentalmaterial das organische Peroxid h), eine Metallverbindung e), ein Halogenid oder Pseudohalogenid f) und einen Initiator c) (Barbitursäurederivate/Malonylsulfamide), so ist es insbesondere sinnvoll, dass das organische Peroxid h), der Initiator c) und die Kombination von Matallverbindung e) und Halogenid bzw. Pseudohalogenid f) in zwei räumlich voneinander getrennten Komponenten vorliegen. Beispielsweise können das organische Peroxid h), die erfindungsgemäßen Reaktiv-Pastenbildner a) und/oder b), ein Initiator c) (Barbitursäurederivate/Malonylsulfamide) sowie Füllstoffe g) zu einer Paste verknetet sein. Andererseits können die polymerisierbaren Monomere d) auch zusammen mit der Kombination von Metallverbindung e) und Halogenid bzw. Pseudohalogenid f) und Füllstoffen g) vorliegen.

Die erfindungsgemäßen Dentalmassen können darüber hinaus einen oder mehrere Zusatzstoffe i) enthalten, die solchen Massen üblicherweise zugesetzt werden. Beispiele dafür sind Puffersalze, Wasserfänger wie z.B. Zeolithe, Carbonsäureanhydride wie z.B. Essigsäureanhydrid, Bernsteinsäureanhydrid, Phthalsäureanhydrid und Maleinsäureanhydrid und/oder entwässerte Salze zur Verhinderung von Hydrolyse- und Umesterungsreaktionen, Metallfänger, wie Quadrapure®, Metallkomplexbildner wie z.B. EDTA, weitere Pastenbildner, Tenside, Wirkstoffe, optische Abtastung ermöglichende Substanzen, Geschmacks- und/oder Geruchsstoffe, Diagnostik ermöglichende Substanzen, Zahnsubstanz (Schmelz, Dentin) ätzende und/oder adhäsiv wirkende Substanzen, Fluoridisierungsmittel, Bleichsubstanzen, Desensibilisierungsmittel, Haftverbundvermittler, Farbstoffe, Farbpigmente, Indikatoren, weitere von Komponenten c), e), f) und h) unterschiedliche Initiatoren oder Initiatorkomponenten wie z.B. anorganische Peroxide, anorganische Persäuren oder deren Perester oder Redoxinitiatorkomponenten und/oder Photoinitiatoren, Stabilisatoren (wie Antioxidantien), Polymerisationsinhibitoren, Thixotropiehilfsmittel sowie antibakterielle Substanzen.

Der Anteil an Inhaltsstoff a) in Komponente A beträgt in der Regel 10-85 Gew. %, bevorzugt 20-80 Gew. %, besonders bevorzugt 30-75 Gew. %.

Der Anteil an Inhaltstoff b) in Komponente A beträgt in der Regel 10-85 Gew. %, bevorzugt 20-80 Gew. %, und besonders bevorzugt 30-75 Gew. %.

Der Anteil an Inhaltstoff c) in Komponente A beträgt in der Regel 0,5-20 Gew. % und bevorzugt 2-10 Gew. %.

Der Anteil an Inhaltstoff h) in Komponente A beträgt in der Regel bis zu 5 Gew. % und bevorzugt bis zu 2 Gew. %.

Der Anteil an Inhaltstoff d) in Komponente B beträgt in der Regel 20-85 Gew. %, bevorzugt 25-80 Gew. % und besonders bevorzugt 30-75 Gew. %.

Der Anteil an Inhaltstoff e) in Komponente B beträgt in der Regel 1-100 ppm und bevorzugt 2-50 ppm.

Der Anteil an Inhaltstoff f) in Komponente B beträgt in der Regel 0,01-1 Gew. % und bevorzugt 0,05-0,5 Gew. %.

Dabei beziehen sich die Gewichtsangaben in den vorangehenden Abschnitten jeweils auf die Gesamtmasse der betreffenden Komponente.

Der Anteil an Inhaltstoff g) in Komponente A beträgt in der Regel 0 bis 80 Gew. %, bezogen auf die Gesamtmasse der Komponente A, vorzugsweise 10 bis 70 Gew.-% und besonders bevorzugt 20 bis 60 Gew.-%; der Anteil an Inhaltstoff g) in Komponente B beträgt in der Regel 10 bis 90 Gew. %, bezogen auf die Gesamtmasse der Komponente B, vorzugsweise 15 bis 70 Gew.-% und besonders bevorzugt 20 bis 60 Gew.-%.

Der Anteil an Inhaltsstoff i) in Komponenten A und/oder B beträgt in der Regel 0 bis 20 Gew. %, bezogen auf die Gesamtmasse der jeweiligen Komponente, vorzugsweise 0 bis 15 Gew.-% und besonders bevorzugt von 0 bis 10 Gew.-%.

Bevorzugt werden polymerisierbare Dentalmaterialien, bei denen Komponente A
- 10 bis 85 Gew.% wenigstens eines Reaktiv-Pastenbildners der Komponente a) und/oder b) enthält, und
- 0,5 bis 20 Gew. % der Komponente c),
wobei die Prozentangaben sich auf die Gesamtmasse der Komponente A beziehen, und bei denen Komponente B
- 20 bis 85 Gew. % an Komponente d),
- 1 bis 100 ppm an Komponente e) und
- 0,01 bis 1 Gew. % an Komponente f) enthält,
wobei die Prozentangaben sich auf die Gesamtmasse der Komponente B beziehen.

Eine Auswahl erfindungsgemäßer Pastenbildner ist in den Ausführungsbeispielen dargestellt.

Vorteilhaft an den alkenylgruppenhaltigen Verbindungen a) und b) ist, dass sie durch die CH-aciden Barbitursäurederivate bzw. Malonylsulfamide während der Lagerung nicht polymerisiert werden, wie dies bei (Meth)acrylaten und Vinylethern (vgl. z.B. DE 100 17 188 B4) der Fall ist; andererseits hingegen nach dem Mischen der erfindungsgemäßen Inhaltsstoffe mit den (Meth)arylsäureestern copolymerisieren und somit gehärtete Produkte mit besseren mechanischen Eigenschaften ausbilden.

Das auf Barbitursäurederivaten und/oder Malonylsulfamiden beruhende Initiatorsystem kann durch weitere Initiatorsysteme ergänzt werden, welche die radikalische Polymerisation der Monomeren d) bewirken. Dabei kann es sich beispielsweise um weitere von Komponenten c), e), f) und h) unterschiedliche Initiatoren oder Initiatorkomponenten, wie z.B. um anorganische Peroxide, Hydroperoxide, Persäuren oder um Redoxinitiatoren oder um Redoxinitiatorkomponenten und/oder um Photoinitiatoren handeln. Durch die Kombination von chemischer und photochemischer Initiierung könnten erfindungsgemäße dualhärtende Dentalmassen hergestellt werden.

Als anorganisches Peroxid eignen sich insbesondere Alkali- oder Erdalkaliperoxodisulfate, insbesondere Natrium- oder Kaliumperoxodisulfat. Als Redoxinitiatorkomponente eignen sich insbesondere Alkali- oder Erdalkalitoluolsulfinate, insbesondere Natrium- oder Kaliumtoluolsulfinat. Diese zusätzlichen Initiatoren können insbesondere bei sauren Formulierungen, welche beispielsweise als selbstätzende Zemente verwendet werden können, eingesetzt werden. In solchen Fällen kann man vorteilhaft das Natriumperoxodisulfat in der Komponente A und das Natriumtoluolsulfinat gegebenenfalls mit basischen Zusatzstoffen in der Komponente B formulieren.

Als Photoinitiatoren eignen sich beispielsweise α-Diketone wie Campherchinon in Verbindung mit sekundären und tertiären Aminen oder Mono- und Bisacylphosphinoxide, wie 2,4,6-Trimethylbenzoyldiphenylphosphinoxid und Bis-(2,6-dichlorbenzoyl)-4-n-propylphenylphosphinoxid. Es eignen sich aber auch andere Verbindungen dieses Typs, wie sie in EP 0 073 413 A2, EP 0 007 508 A2, EP 0 047 902 A2, EP 0 057 474 A2 und EP 0 184 095 A2 beschrieben sind.

Es hat sich überraschenderweise herausgestellt, dass die reaktiven, ausgewählte Alkenylgruppen enthaltenden Reaktiv-Pastenbildner a) und/oder b) mit Barbitursäurederivaten und/oder Malonylsulfamiden als Initiatorkomponente A formuliert werden können, ohne dass eine vorzeitige Polymerisation der Alkenylgruppen stattfindet, wie aus dem eingangs zitierten Stand der Technik zu erwarten gewesen wäre.

Es wird erfindungsgemäß vorgeschlagen, dass Komponente A und Komponente B jeweils als Paste und räumlich voneinander getrennt vorliegen.

Die Verwendung räumlich voneinander getrennter Pasten, d.h. von Initiatorpaste (Komponente A) und von Basispaste (Komponente B), verhindert während der Lagerung die vorzeitige Bildung von Radikalen und damit die verfrüht einsetzende Polymerisation der beiden Komponenten. Zudem sind Pasten für die Handhabung des polymerisierbaren Dentalmaterials von Vorteil, da diese sowohl von Hand als auch durch selbst anmischende Systeme (z.B. Doppelkartusche mit statischen oder dynamischen Mischkanülen) sorgfältiger vermischt werden können, als dies beispielsweise bei auf Pulver und Flüssigkeit beruhenden Mehrkomponenten-Systemen der Fall ist.

Die Komponente A des polymerisierbaren Dentalmaterials gemäß der vorliegenden Erfindung ist sowohl bei Raumtemperatur als auch bei Stresstemperaturen von 40 °C und zum Teil auch bei 60° C lagerstabil, d.h. die reaktiven, alkenylgruppenhaltigen Reaktiv-Pastenbildner a) und/oder b) (Monomere, Oligomere, Polymere) können mit Barbitursäurederivaten und/oder Malonylsulfamiden die Initiatorpaste bilden, ohne dass unter Lagerbedingungen eine vorzeitige Polymerisation der Alkenylgruppen stattfindet. Eine gleich gute Lagerstabilität lässt sich auch für die Komponente B des polymerisierbaren Dentalmaterials feststellen.

Die erfindungsgemäße Mehrkomponentenzusammensetzung wird durch Vermischen der einzelnen Komponenten des zuvor beschriebenen Dentalmaterials zu einer polymerisationsfähigen Dentalmasse verarbeitet. Vorzugsweise wird eine Basiskomponente B mit einer Katalysatorkomponente A in einem Verhältnis von 1:2 bis 20:1, besonders bevorzugt von 1:1 bis 10:1 und ganz besonders bevorzugt von 10:1, 5:1, 4:1, 2:1 und 1:1, vermischt. Diese Mischungen zeichnen sich durch eine rasche Polymerisation bei Mundbedingungen aus.

Nach dem Vermischen der Initiatorpaste A mit der Komponente B (Basispaste) copolymerisieren die alkenylgruppenhaltigen Reaktiv-Pastenbildner mit den in der Basispaste enthaltenen Acrylat- und/oder (Meth)acrylat-Monomeren bzw. Oligo-Polymeren radikalisch bei Bedingungen, die denen im Mund eines Patienten entsprechen (Temperatur ∼ 35°C, rel. Luftfeuchte ∼ 100 %). Dabei werden die alkenylgruppenhaltigen Pastenbildner in das entstehende Netzwerk mit eingebunden; dieses führt zu verbesserten mechanischen Eigenschaften des ausgehärteten Polymerisats.

Das erfindungsgemäße Mehrkomponentensystem wird vorzugsweise in geeigneten Primärverpackungen, wie Tuben, Dosen, und besonders bevorzugt in Kartuschen und Schlauchbeuteln, wie sie z.B. in der EP-A-723,807, der EP-A-541,972, der WO 98/44860 A1, der EP-A-492,412, der EP-A-492,413 und der EP-A-956,908 beschrieben sind, gelagert und ist auf die spätere Verwendung zugeschnitten proportioniert.

Eine spezielle Ausführungsform betrifft die Verwendung des vorstehend beschriebenen polymerisierbaren Dentalmaterials als Befestigungsmaterial, Bondingmaterial, Stumpfaufbaumaterial, zahntechnischen Werkstoff zur Herstellung von Inlays, von Onlays, von Verblendschalen und von künstlichen Zähnen, als Modellmaterial, Dentalzement sowie als provisorisches und permanentes Kronen- und Brückenmaterial. Ganz besonders bevorzugt wird das erfindungsgemäße Dentalmaterial zur Herstellung von Kronen- und Brückenmaterial verwendet, wobei hierunter temporärer und dauerhafter provisorischer und definitiver Zahnersatz zu verstehen ist. Ganz besonders bevorzugt wird das erfindungsgemäßen Dentalmaterial als Dentalzement eingesetzt, insbesondere als selbstätzender Dentalzement.

Die Erfindung betrifft auch ein gehärtetes Dentalmaterial, das erhältlich ist durch Vermischen der oben beschriebenen Komponenten A und B, vorzugsweise im Verhältnis 1: 20 bis 1: 1, und durch Polymerisation der dadurch erhaltenen polymerisierbaren Dentalmaterials.

Weiterbildungen, Vorteile und Anwendungsmöglichkeiten der Erfindung ergeben sich auch aus der nachfolgenden Beschreibung von bevorzugten Ausführungsbeispielen. Dabei bilden alle beschriebenen Merkmale für sich oder in beliebiger sinnvoller Kombination den Gegenstand der Erfindung, auch unabhängig von ihrer Zusammenfassung in einzelnen Ansprüchen und/oder deren Rückbeziehung.

Die Prozentangaben in den vorliegenden Unterlagen werden als Gewichtsprozent (Gew.-%) definiert, sofern nichts anderes angegeben ist.

Polymerisierte Dentalmaterialien für die oben genannten Anwendungen erfordern eine hohe Festigkeit, da die erfindungsgemäßen gehärteten Dentalprodukte beispielsweise aufgrund der auftretenden Beißkräfte auf der Okklusion sowohl sehr bruchstabil sein müssen als auch die Relation der Zahnstümpfe ausreichend stabilisieren sollen. Durch das Einbinden aller Inhaltsstoffe in das polymerisierte Dentalmaterial werden signifikant bessere mechanische Eigenschaften erreicht, wie nachstehend aus Tabelle 18 deutlich wird.

### Beispiele

Nachfolgend sind die Strukturen der in den erfindungsgemäßen Formulierungen eingesetzten Verbindungen 1a, 2a, 2b, 3a, 4a und der in den nicht erfindungsgemäßen Formulierungen (= Vergleichsbeispiele) eingesetzten Verbindungen 1b, 2c, 2d, 2e, 3b, 4b dargestellt.

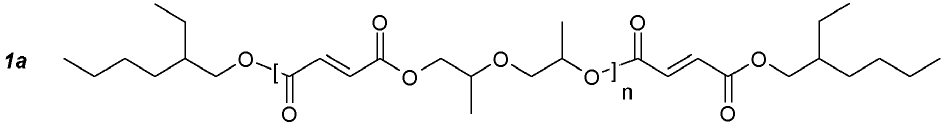

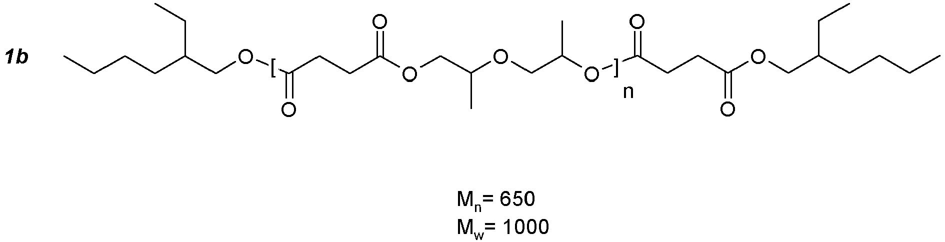

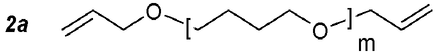

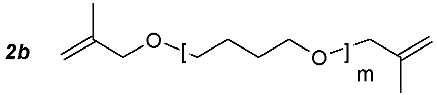

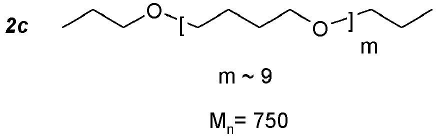

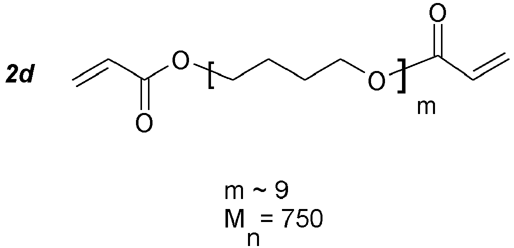

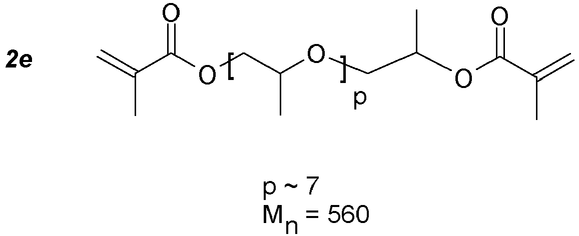

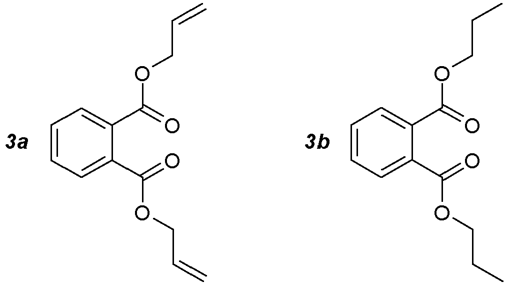

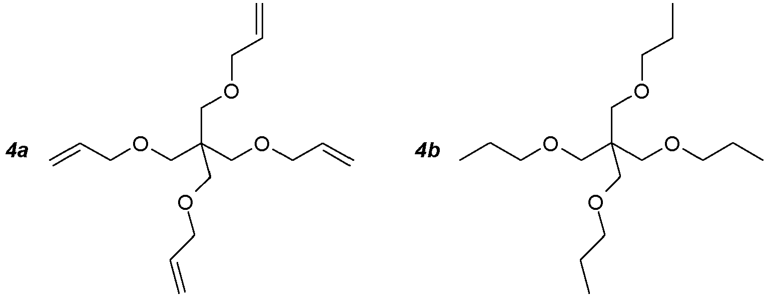

### Herstellung der Ausgangsmaterialien

### Hydrierung von 1a, 2a, und 4a:

100 ml der zu hydrierenden Substanz wurden in ca. 100 ml Diethylether (abs.) gelöst und in einen 350 ml Stahlautoklaven überführt. Nach Zugabe von ca. 5 g Pd/C-Katalysator (10% Pd) wurde mit 5 bar Wasserstoffdruck bei 60 °C hydriert. Zur Kontrolle des Reaktionsfortschrittes wurde der Autoklav von der Wasserstoffversorgung getrennt und beobachtet, ob weiterer Wasserstoff aufgenommen wurde (bei Druckabfall wurde weiter hydriert). Nach 24 Stunden wurde kein weiterer Wasserstoff aufgenommen. Nach Abfiltrieren des Pd/C-Katalysators wurde das Produkt eingeengt und NMR-spektroskopisch untersucht. Alle NMR-Spektren wurden bei Raumtemperatur mit einem Bruker DRX-250 Spektrometer (¹H-NMR bei 250 MHz, ¹³C-NMR bei 62 MHz) aufgenommen. Chemische Verschiebungen in ppm sind auf das jeweilige Lösungsmittelsignal (¹H-NMR, ¹³C-NMR) bezogen.

### Verbindung 1b:

**¹H NMR (CDCl₃, 250 MHz):** δ 4,91-5,16 (m, CH(CH₃)OC(O)), 3,82-4,12 (m, OCH₂-CH(Et) + OCH₂CH(CH₃)O), 3,58-3,82 (m, OCH₂CH(Me)O), 3,33-3,58 (m, OCH₂CH(Me)OC(O)), 2,40-2,73 (m, C(O)CH₂CH₂C(O)), 1,42-1,62 (m, CH(Et)), 1,01-1,40 (m, CH(CH₃)) + CH(CH₂CH₃)CH₂CH₂CH₂Me), 0,73-0,96 (m, CH₃CH₂CH₂).

¹³C **NMR (CDCl₃, 62 MHz):** δ 172,74 (OCH(Me)OC(O)), 172,72 (CH(Et)CH₂OC(O)), 172.06 (CH₂CH₂C(O)OCH₂),73,84 (CH₂CH(Me)O), 71.93 (OCH₂CH(Me)OC(O)), 69,94 (OCH₂CH(Me)O), 67,78 (C(O)OCH₂CH(Me)O), 67,47 (CH(Et)CH₂O), 30,69 (CH(Et)CH₂CH₂), 29,71 (CH(Me)OC(O)CH₂CH₂), 29,58 (CH(Et)CH₂OC(O)CH₂), 29,44 (CH(Et)CH₂OC(O)CH₂CH₂), 29,40 (CH(Me)OC(O)CH₂CH₂), 29,24 (MeCH₂CH₂CH₂), 24,07 (CH(CH₂CH₃), 23,28 (MeCH₂CH₂CH₂), 17,27 (MeCHOCH₂), 16,88 (MeCHOC(O)), 14.35 (MeCH₂CH₂CH₂), 11,29 (MeCH₂CH).

### Verbindung 2c:

**¹H NMR (CDCl₃, 250 MHz):** δ 3,18-3.37 (m, OCH₂), 1,39-1,57 (m, C-CH₂), 0,78 (t, J=7,41, CH₃).

¹³C **NMR (CDCl₃, 62 MHz):** δ 72,85 (OCH₂CH₂CH₃), 70,91 (br, OCH₂CH₂CH₂CH₂O), 26,85 (OCH₂CH₂CH₂CH₂O), 23,27 (OCH₂CH₂CH₃), 10,93 (Me).

### Verbindung 4b:

**¹H NMR (DMSO, 250 MHz):** δ 3,22-3,31 (t, OCH₂CH₂), 1,38-1,56 (m, CH₂-CH₂-CH₃), 0,78-0,89 (t, CH₃).

**¹³C NMR (DMSO, 62 MHz):** δ 72,61 (OCH₂CH₂CH₃), 69,45 (CCH₂), 45,65 (C), 22,72 (CH₂CH₃), 10,77 (CH₃).

Zur Herstellung der erfindungsgemäßen Initiatorpasten wurden die in den nachstehenden Tabellen 1, 3, 4, 8, 10, 12, 14 und 15 aufgeführten Inhaltsstoffe homogenisiert und über ein Dreiwalzwerk dispergiert. Anschließend wurden die Initiatorpasten in die kleine Kammer einer 10:1 Kartusche Typ Mixpac CS 050-10-06 (Sulzer) abgefüllt, in die große Kammer wurde die auf die gleiche Weise hergestellte Basispaste nach Tabelle 17 gegeben. Die Massen wurden durch Zentrifugieren entgast, die Kartuschen wurden verschlossen und die Dentalmassen wurden über einen statischen Mischer Typ Mixpac MBX 3.2-16-S (Sulzer) ausgetragen. Zum Vergleich wurden die in den Tabellen 2, 5, 6, 7, 9, 11, 13 und 16 aufgeführten Inhaltsstoffe zu nicht erfindungsgemäßen Initiatorpasten in der gleichen Art und Weise verarbeitet.

Der E-Modul und die Biegefestigkeit wurden in Anlehnung an EN ISO 4049 bestimmt (mindestens 8 Prüfkörper pro Messung). Die Ergebnisse sind in Tabelle 18 dargestellt.

**Tabelle 1: Herstellungsbeispiel Initiatorpaste Ia (erfindungsgemäß)**

| **Inhaltsstoff** | **Menge [%]** | **Menge [g]** |
|---|---|---|
| ungesättigtes Polyesterharz ***1a*** | 47,00 | 9,40 |
| Bariumglaspulver, unsilanisiert 1,5 µm | 41,50 | 8,30 |
| 1-Benzyl-5-phenylbarbitursäure | 8,00 | 1,60 |
| HDK® H2000 ¹⁾ | 3,50 | 0,70 |

**Tabelle 2: Herstellungsbeispiel Initiatorpaste Ib (Vergleich, nicht erfindungsgemäß)**

| **Inhaltsstoff** | **Menge [%]** | **Menge [g]** |
|---|---|---|
| gesättigtes Polyesterharz ***1b*** | 47,00 | 9,40 |
| Bariumglaspulver, unsilanisiert 1,5 µm | 41,50 | 8,30 |
| 1-Benzyl-5-phenylbarbitursäure | 8,00 | 1,60 |
| HDK® H2000 ¹⁾ | 3,50 | 0,70 |

**Tabelle 3: Herstellungsbeispiel Initiatorpaste IIa (erfindungsgemäß)**

| **Inhaltsstoff** | **Menge [%]** | **Menge [g]** |
|---|---|---|
| Poly-THF-diallylether ***2a*** | 40,00 | 8,00 |
| Bariumglaspulver, unsilanisiert 1,5 µm | 48,50 | 9,70 |
| 1-Benzyl-5-phenylbarbitursäure | 8,00 | 1,60 |
| HDK® H2000 ¹⁾ | 3,50 | 0,70 |

**Tabelle 4: Herstellungsbeispiel Initiatorpaste IIb (erfindungsgemäß)**

| **Inhaltsstoff** | **Menge [%]** | **Menge [g]** |
|---|---|---|
| Poly-THF-dimethallylether ***2b*** | 40,00 | 8,00 |
| Bariumglaspulver, unsilanisiert 1,5 µm | 48,50 | 9,70 |
| 1-Benzyl-5-phenylbarbitursäure | 8,00 | 1,60 |
| HDK® H2000 ¹⁾ | 3,50 | 0,70 |

**Tabelle 5: Herstellungsbeispiel Initiatorpaste IIc (Vergleich, nicht erfindungsgemäß)**

| **Inhaltsstoff** | **Menge [%]** | **Menge [g]** |
|---|---|---|
| Poly-THF-dipropylether ***2c*** | 40,00 | 8,00 |
| Bariumglaspulver, unsilanisiert 1,5 µm | 48,50 | 9,70 |
| 1-Benzyl-5-phenylbarbitursäure | 8,00 | 1,60 |
| HDK® H2000 ¹⁾ | 3,50 | 0,70 |

**Tabelle 6: Herstellungsbeispiel Initiatorpaste IId (Vergleich, nicht erfindungsgemäß)**

| **Inhaltsstoff** | **Menge [%]** | **Menge [g]** |
|---|---|---|
| Poly-THF-diacrylat ***2d*** | 40,00 | 8,00 |
| Bariumglaspulver, unsilanisiert 1,5 µm | 48,50 | 9,70 |
| 1-Benzyl-5-phenylbarbitursäure | 8,00 | 1,60 |
| HDK® H2000 ¹⁾ | 3,50 | 0,70 |

**Tabelle 7: Herstellungsbeispiel Initiatorpaste IIe (Vergleich, nicht erfindungsgemäß)**

| **Inhaltsstoff** | **Menge [%]** | **Menge [g]** |
|---|---|---|
| Poly(propylenglycol)-dimethacrylat ***2e*** | 40,00 | 8,00 |
| Bariumglaspulver, unsilanisiert 1,5 µm | 48,50 | 9,70 |
| 1-Benzyl-5-phenylbarbitursäure | 8,00 | 1,60 |
| HDK® H2000 ¹⁾ | 3,50 | 0,70 |

**Tabelle 8: Herstellungsbeispiel Initiatorpaste IIIa (erfindungsgemäß)**

| **Inhaltsstoff** | **Menge [%]** | **Menge [g]** |
|---|---|---|
| Diallylphthlat ***3a*** | 41,00 | 8,20 |
| Bariumglaspulver, unsilanisiert 1,5 µm | 47,50 | 9,50 |
| 1-Benzyl-5-phenylbarbitursäure | 8,00 | 1,60 |
| HDK® H2000 ¹⁾ | 3,50 | 0,70 |

**Tabelle 9: Herstellungsbeispiel Initiatorpaste IIIb (Vergleich, nicht erfindungsgemäß)**

| **Inhaltsstoff** | **Menge [Gew.-%]** | **Menge [g]** |
|---|---|---|
| Dipropylphthalat ***3b*** | 41,00 | 8,20 |
| Bariumglaspulver, unsilanisiert 1,5 µm | 47,50 | 9,50 |
| 1-Benzyl-5-phenylbarbitursäure | 8,00 | 1,60 |
| HDK® H2000 ¹⁾ | 3,50 | 0,70 |

**Tabelle 10: Herstellungsbeispiel Initiatorpaste IVa (erfindungsgemäß)**

| **Inhaltsstoff** | **Menge [%]** | **Menge [g]** |
|---|---|---|
| Tetraallylpentaerytriol ***4a*** | 41,00 | 8,20 |
| Bariumglaspulver, unsilanisiert 1,5 µm | 47,50 | 9,50 |
| 1-Benzyl-5-phenylbarbitursäure | 8,00 | 1,60 |
| HDK® H2000 ¹⁾ | 3,50 | 0,70 |

**Tabelle 11: Herstellungsbeispiel Initiatorpaste IVb (Vergleich, nicht erfindungsgemäß)**

| **Inhaltsstoff** | **Menge [%]** | **Menge [g]** |
|---|---|---|
| Tetrapropylpentaerytriol ***4b*** | 41,00 | 8,20 |
| Bariumglaspulver, unsilanisiert 1,5 µm | 47,50 | 9,50 |
| 1-Benzyl-5-phenylbarbitursäure | 8,00 | 1,60 |
| HDK® H2000 ¹⁾ | 3,50 | 0,70 |

**Tabelle 12: Herstellungsbeispiel Initiatorpaste Va mit Perester (erfindungsgemäß)**

| **Inhaltsstoff** | **Menge [%]** | **Menge [g]** |
|---|---|---|
| ungesättigtes Polyesterharz ***1a*** | 46,00 | 4,60 |
| Bariumglaspulver, unsilanisiert 1,5 µm | 41,50 | 4,15 |
| 1-Benzyl-5-phenylbarbitursäure | 8,00 | 0,80 |
| HDK H2000 ¹⁾ | 3,50 | 0,35 |
| *tert*.-Butylperoxy-3,5,5-trimethylhexanoat | 1,00 | 0,10 |

**Tabelle 13: Herstellungsbeispiel Initiatorpaste Vb mit Perester (Vergleich, nicht erfindungsgemäß)**

| **Inhaltsstoff** | **Menge [%]** | **Menge [g]** |
|---|---|---|
| gesättigtes Polyesterharz ***1b*** | 46,00 | 4,60 |
| Bariumglaspulver, unsilanisiert 1,5 µm | 41,50 | 4,15 |
| 1-Benzyl-5-phenylbarbitursäure | 8,00 | 0,80 |
| HDK® H2000 ¹⁾ | 3,50 | 0,35 |
| *tert*.-Butylperoxy-3,5,5-trimethylhexanoat | 1,00 | 0,10 |

**Tabelle 14: Herstellungsbeispiel Initiatorpaste Vla mit Perester (erfindungsgemäß)**

| **Inhaltsstoff** | **Menge [%]** | **Menge [g]** |
|---|---|---|
| Poly-THF-diallylether ***2a*** | 39,00 | 3,90 |
| Bariumglaspulver, unsilanisiert 1,5 µm | 48,50 | 4,85 |
| 1-Benzyl-5-phenylbarbitursäure | 8,00 | 0,80 |
| HDK® H2000 ¹⁾ | 3,50 | 0,35 |
| *tert*.-Butylperoxy-3,5,5-trimethylhexanoat | 1,00 | 0,10 |

**Tabelle 15: Herstellungsbeispiel Initiatorpaste VIb mit Perester (erfindungsgemäß)**

| **Inhaltsstoff** | **Menge [%]** | **Menge [g]** |
|---|---|---|
| Poly-THF-dimethallylether ***2b*** | 39,00 | 3,90 |
| Bariumglaspulver, unsilanisiert 1,5 µm | 48,50 | 4,85 |
| 1-Benzyl-5-phenylbarbitursäure | 8,00 | 0,80 |
| HDK® H2000 ¹⁾ | 3,50 | 0,35 |
| *tert*.-Butylperoxy-3,5,5-trimethylhexanoat | 1,00 | 0,10 |

**Tabelle 16: Herstellungsbeispiel Initiatorpaste VIc mit Perester (Vergleich, nicht erfindungsgemäß)**

| **Inhaltsstoff** | **Menge [%]** | **Menge [g]** |
|---|---|---|
| Poly-THF-dipropylether ***2*c** | 39,00 | 3,90 |
| Bariumglaspulver, unsilanisiert 1,5 µm | 48,50 | 4,85 |
| 1-Benzyl-5-phenylbarbitursäure | 8,00 | 0,80 |
| HDK® H2000 ¹⁾ | 3,50 | 0,35 |
| *tert*.-Butylperoxy-3,5,5-trimethylhexanoat | 1,00 | 0,10 |

**Tabelle 17: Herstellungsbeispiel VII Basispaste**

| **Inhaltsstoff** | **Menge [%]** | **Menge [g]** |
|---|---|---|
| ethoxyliertes Bisphenol-A-di(meth)acrylat (4EO) | 42,50 | 85,00 |
| aliphatisches Urethandi(meth)acrylat | 13,50 | 27,00 |
| Bariumglaspulver 1,5 µm, (meth)acryl-silanisiert | 36,58 | 73,16 |
| Aerosil DT4 ²⁾ | 7,00 | 14,00 |
| 4-Hydroxyanisol | 0,10 | 0,20 |
| Kupfer(II)di(meth)acrylat in Hydroxyethyl-(Meth)acrylat (1 % Lsg.) | 0,12 | 0,24 |
| Dodecyltrimethylammoniumchlorid | 0,20 | 0,40 |

| | | |
|---|---|---|
| ¹⁾ Bei dem Inhaltsstoff HDK® H2000 handelt es sich um eine mit Trimethylsiloxygruppen oberflächenmodifizierte pyrogene Kieselsäure mit einem Kohlenstoffgehalt von 2,5 % und einer spezifischen BET-Oberfläche von 140 m²/g (nach DIN 66131 und DIN 66132), die unter diesem Namen bei Wacker-Chemie GmbH, München, D erhältlich ist. ²⁾ Bei dem Inhaltsstoff Aerosil DT4 handelt es sich um ein Methacryl-silanisiertes hydrophobes Siliziumdioxid, das unter diesem Namen bei der Evonik Degussa GmbH, Frankfurt a.M., D erhältlich ist. | | |

**Tabelle 18: Ergebnisse von gehärteten Produkten aus Abmischungen aus Basispaste nach Tabelle 17 und verschiedenen Initiatorpasten**

| Initiatorpaste Nr. | E-ModuI¹⁾ [MPa] | Biegefest.¹⁾ [MPa] |
|---|---|---|
| ***Ia*** | 3391±87 | 104±4 |
| ***Ib*** | 3107±147 | 94±5 |
| ***IIa*** | 3775±158 | 109±7 |
| ***IIb*** | 3411±143 | 100±1 |
| ***IIc*** | 2901±117 | 84±3 |
| ***IId*** | ²⁾ | ²⁾ |
| ***IIe*** | ²⁾ | ²⁾ |
| ***IIIa*** | 4034±184 | 111±6 |
| ***IIIb*** | 3268±143 | 95±4 |
| ***IVa*** | 4023±100 | 109±5 |
| ***IVb*** | 2981±135 | 88±4 |
| ***Va*** | 4963±70 | 139±3 |
| ***Vb*** | 4627±107 | 130±6 |
| ***VIa*** | 4736±168 | 134±6 |
| ***VIb*** | 5160±265 | 145±3 |
| ***VIc*** | 4381±158 | 120±7 |

| | | |
|---|---|---|
| ¹⁾ Abweichung als σ (Standardabweichung) ²⁾ die Initiatorpaste härtete als Einzelkomponente vorzeitig aus und war deshalb nicht lagerstabil | | |

Aus Tabelle 18 ist zu erkennen, dass im Vergleich zu den bekannten Dentalmaterialien mit nicht reaktiven Pastenbildnern (d.h. ohne Alkenylgruppen), wie sie im Stand der Technik beschrieben werden, die polymerisierbaren Dentalmaterialien mit Reaktiv-Pastenbildnern gemäß der vorliegenden Erfindung signifikant bessere mechanische Eigenschaften nach der Polymerisation im Endprodukt erzielen. Dies wird beispielsweise durch höhere Biegefestigkeiten und E-Moduli im Dreipunktbiegeversuch dokumentiert.

Zur Untersuchung der Lagerstabilität wurden die Kartuschen Ia, Ib, IIa, IIb, IIc, IId, IIe, IIIa, IIIb, IVa und IVb bei 60°C, 37°C, und Raumtemperatur eingelagert. Während der Inhalt aller Kartuschen Ia, Ib, IIa, IIb, IIc, IIIa, IIIb, IVa und IVb nach einer Lagerzeit von 4 Wochen pastös war und nach dem Austragen über einen statischen Mischer in der erwarteten Zeit aushärtete, war bei der Kartusche IId und IIe eine vorzeitige Aushärtung des Materials zu beobachten. Der Inhalt der bei 60°C eingelagerten Kartusche war bereits nach 3 Stunden (IId) bzw. nach 1 Stunde (IIe) ausgehärtet, der bei 37°C eingelagerten Kartusche nach 24 Stunden (IId) bzw. nach 5 Stunden (IIe) und der bei Raumtemperatur gelagerten Kartusche nach 2 Tagen (IId) bzw. nach 22 Stunden (IIe).

Die Kartuschen Va, Vb, VIa, VIb und VIc mit Perestern als Inhaltsstoffen wurden bei 37°C und Raumtemperatur eingelagert. In allen Fällen war der Inhalt nach einer Lagerzeit von 4 Wochen pastös und härtete nach dem Austragen über einen statischen Mischer in der erwarteten Zeit aus. Die erfindungsgemäßen Formulierungen zeigten signifikant bessere mechanische Eigenschaften.

## Patentansprüche

1. Polymerisierbares Dentalmaterial enthaltend wenigstens eine pastenförmige Komponente A und wenigstens eine pastenförmige Komponente B, wobei Komponente A mindestens einen Initiator der radikalischen Polymerisation c) ausgewählt aus der Gruppe der Barbitursäurederivate und/oder der Malonylsulfamide enthält und wobei Komponente B mindestens eine Acrylsäureester- und/oder Methacrylsäureesterreste umfassende organische Verbindung d), mindestens eine Metallverbindung e) und mindestens eine Halogenid- und/oder Pseudohalogenidverbindung f) enthält, wobei Inhaltstoff e) eine Metallverbindung ist, die ausgewählt wird aus der Gruppe der der Salze der Metalle der dritten und vierten Hauptgruppe sowie der ersten bis achten Nebengruppe des Periodensystems der Elemente einschließlich der Lanthaniden und/oder der Metallkomplexe, wobei Inhaltstoff f) ein Halogenid oder ein Pseudohalogenid ist mit Metallkationen von Metallen der ersten und der zweiten Hauptgruppe des Periodensystems, Ammonium- oder Phosphoniumkationen, **dadurch gekennzeichnet, dass** Komponente A als Reaktiv-Pastenbildner mindestens eine von Maleinsäure und/oder von Fumarsäure abgeleitete organische Verbindung a), die neben den von Maleinsäure und/oder von Fumarsäure abgeleiteten Gruppen keine weiteren ethylenisch ungesättigten Gruppen aufweist, und/oder mindestens eine Verbindung b) umfassend mindestens einen Allyl- und/oder Methallylrest und gegebenenfalls von Maleinsäure und/oder Fumarsäure abgeleitete Einheiten, die neben den vorstehend genannten ethylenisch ungesättigten Gruppen keine weiteren ethylenisch ungesättigten Gruppen aufweist, enthält.

2. Polymerisierbares Dentalmaterial nach Anspruch 1, **dadurch gekennzeichnet, dass** Inhaltstoff a) mindestens eine organische Verbindung enthält, die Maleinsäurediester- und/oder Fumarsäurediesterreste aufweist oder die Maleinsäurediamid- und/oder Fumarsäurediamidreste aufweist, insbesondere eine organische Verbindung der Formeln Ia, Ib, IIa, IIb und insbesondere der Formeln IIc oder IId 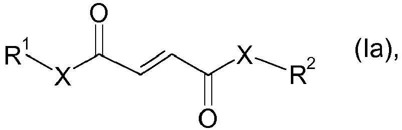 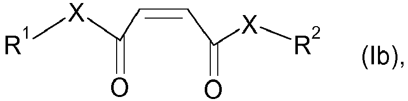 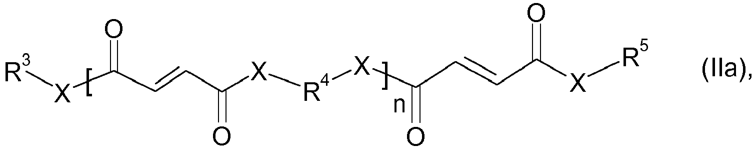 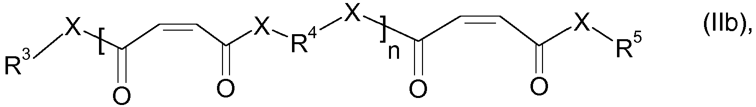 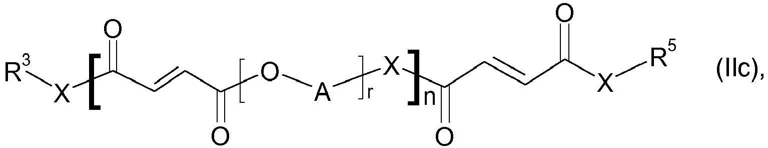 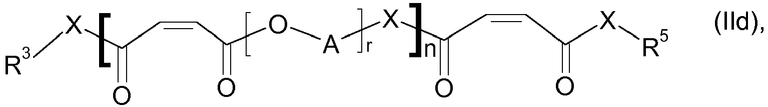
worin X Sauerstoff oder eine Gruppe -NR⁶- bedeutet,
R¹, R², R³ und R⁵ unabhängig voneinander Alkyl, Cycloalkyl, Alkylcycloalkyl, Aryl, Alkylaryl, Aralkyl oder Heterocyclyl bedeuten, die gegebenenfalls einen oder mehrere Substituenten aufweisen,
R⁴ Alkylen, Alkylenglykolether, Cycloalkylen, Alkylcycloalkylen, Arylen, Alkylarylen, Aralkylen oder Heterocyclylen bedeutet, das gegebenenfalls einen oder mehrere Substituenten aufweist,
A = CH₂-CH₂, CH₂-CH(CH₃) oder CH₂-CH₂-CH₂-CH₂ bedeutet,
R⁶ Wasserstoff, Alkyl, Cycloalkyl, Alkylcycloalkyl, Aryl, Alkylaryl, Aralkyl oder Heterocyclyl ist,
n eine ganze Zahl von 1 bis 50 ist, und
r eine ganze Zahl von 1 bis 100 bedeutet.

3. Polymerisierbares Dentalmaterial nach Anspruch 2, **dadurch gekennzeichnet, dass** ein oder mehrere der Reste R¹ bis R⁵ Substituenten mit Säurefunktionen aufweisen, insbesondere Phosphorsäuregruppen, Phosphonsäuregruppen, Sulfonsäuregruppen und/oder Carbonsäure-gruppen sowie deren Anhydride, oder dass ein oder mehrere der Reste R¹ bis R⁵ Substituenten mit Säurefunktionen und Hydroxylgruppen als Substituenten aufweisen oder dass als Inhaltsstoff a) Gemische von säure- und hydroxyfunktionalisierten Verbindungen der Formeln Ia, Ib, IIa, IIb, IIc und/oder IId eingesetzt werden.

4. Polymerisierbares Dentalmaterial nach einem der Ansprüche 1 bis 3, **dadurch gekennzeichnet, dass** Inhaltstoff b) eine Verbindung ist, die Allylether- und/oder Methallyletherreste enthält und die darüber hinaus keine weiteren ethylenisch ungesättigten Gruppen aufweist, insbesondere eine Verbindung der Formel III
R⁷-[-Y-R⁸]ₘ (III),
worin R⁷ ein m-wertiger Rest ist, der gegebenenfalls einen oder mehrere Substituenten aufweist,
R⁸ eine Gruppe -CH₂-CR⁹=CH₂ ist,
R⁹ Wasserstoff oder Methyl bedeutet,
m eine ganze Zahl von 1 bis 12 ist, und
Y ausgewählt wird aus der Gruppe der kovalenten Bindung oder einem zweiwertigen Rest.

5. Polymerisierbares Dentalmaterial nach Anspruch 4, **dadurch gekennzeichnet, dass** Rest R⁷ Substituenten mit Säurefunktionen aufweist, insbesondere Phosphorsäuregruppen, Phosphonsäuregruppen, Sulfonsäuregruppen und/oder Carbonsäuregruppen sowie deren Anhydride, oder dass Rest R⁷ Substituenten mit Säurefunktionen und Hydroxylgruppen als Substituenten aufweist oder dass als Inhaltsstoff b) Gemische von säure- und hydroxyfunktionalisierten Verbindungen der Formel III eingesetzt werden.

6. Polymerisierbares Dentalmaterial nach einem der Ansprüche 1 bis 5, **dadurch gekennzeichnet, dass** Inhaltstoff c) ausgewählt wird aus der Gruppe der Verbindungen der Formel IV, V oder VI 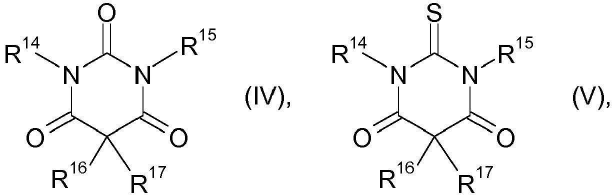 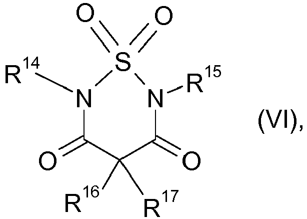
worin R¹⁶ und R¹⁷ unabhängig voneinander Wasserstoff, Alkyl, Alkenyl, Cycloalkyl, Aryl, Aralkyl und Heterocyclyl bedeuten, mit der Maßgabe, dass mindestens einer der Reste R¹⁶ oder R¹⁷ Wasserstoff bedeutet, und
R¹⁴ und R¹⁵ unabhängig voneinander Wasserstoff, Alkyl, Alkenyl, Cycloalkyl, Aryl, Aralkyl und Heterocyclyl bedeuten, insbesondere aus der Gruppe der Barbitursäurederivate der Formel IV, worin R¹⁴ und/oder R¹⁶ oder R¹⁷ unabhängig voneinander Alkyl, Cycloalkyl, Aryl oder Aralkyl bedeuten.

7. Polymerisierbares Dentalmaterial nach einem der Ansprüche 1 bis 6, **dadurch gekennzeichnet, dass** Inhaltstoff d) eine organische Verbindung der Formel VII ist, 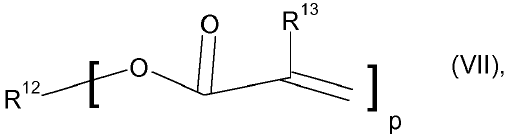 worin R¹² ein p-wertiger organischer Rest ist, der gegebenenfalls einen oder mehrere Substituenten aufweist, R¹³ Wasserstoff oder Methyl bedeutet, und p eine ganze Zahl von 1 bis 12 ist.

8. Polymerisierbares Dentalmaterial nach einem der Ansprüche 1 bis 7, **dadurch gekennzeichnet, dass** Inhaltstoff e) Anionen abgeleitet von Carbonsäuren, insbesondere der Acrylsäure oder der Methacrylsäure, besonders bevorzugt Salze des Kupfers, Eisens, Zinns, Chroms, Mangans, Kobalts, Zinks, Nickels, der Seltenen Erden und des Aluminiums, oder der Komplexe von Metallen der dritten und vierten Hauptgruppe sowie der ersten bis achten Nebengruppe des Periodensystems der Elemente einschließlich der Lanthaniden mit Liganden abgeleitet von Acetylacetonat, besonders bevorzugt Komplexe des Kupfers, Eisens, Zinns, Chroms, Mangans, Kobalts, Zinks, Nickels, der Seltenen Erden und des Aluminiums, und worin Inhaltsstoff e) ganz besonders bevorzugt eine Kupferverbindung ist, insbesondere Kupferacetylacetonat, Kupfernaphthenat, Kupfermethacrylat, Kupferacrylat, Kupferacetat, Kupferoleat, Kupferethylhexanoat oder Kupfercylohexylbutyrat umfasst.

9. Polymerisierbares Dentalmaterial nach einem der Ansprüche 1 bis 8, **dadurch gekennzeichnet, dass** Inhaltstoff f) Ammonium- oder Phosphoniumkationen mit organischen Resten einschließlich der Hydrohalogenide von Aminen umfasst, ganz besonders bevorzugt ein Chlorid, Bromid oder lodid, das als Kation ein Metallkation, insbesondere ein Lithiumkation oder ein Natriumkation, oder ein Ammoniumkation oder ein Phosphoniumkation aufweist oder ein Thiocyanat, Isothiocyanat, Cyanat oder Isocyanat, das als Kation ein Lithiumkation oder ein Natriumkation oder ein Ammoniumkation oder ein Phosphoniumkation aufweist oder ein Hydrohalogenid eines tertiären Amins, und worin Inhaltsstoff f) ganz besonders bevorzugt aus Lithium-, Ammonium- oder Phosphoniumhalogeniden oder deren Hydrohalogeniden ausgewählt wird.

10. Polymerisierbares Dentalmaterial nach einem der Ansprüche 1 bis 9, **dadurch gekennzeichnet, dass** Komponente A und/oder B zusätzlich mindestens einen Füllstoff g) enthält, der vorzugsweise in Komponente A in einer Menge von 0 bis 80 Gew. % und in Komponente B in einer Menge von 10 bis 90 Gew. %, bezogen auf die Gesamtmasse der jeweiligen Komponente, vorliegt.

11. Polymerisierbares Dentalmaterial nach einem der Ansprüche 1 bis 10, **dadurch gekennzeichnet, dass** Komponente A und/oder B zusätzlich mindestens ein organisches Peroxid h) enthält, das vorzugsweise in einer Menge von bis zu 5 Gew. %, bezogen auf die Gesamtmasse der Komponente A und/oder B, vorliegt, wobei Komponente h) besonders bevorzugt ausgewählt wird aus der Gruppe der Carbonsäure- und/oder Kohlensäure-Peroxyester und/oder Perketale, ganz besonders bevorzugt aus der Gruppe tert-Butylperoxy-3,5,5-trimethyl-hexanoat, tert-Butylperoxy-benzoat, tert-Butylperoxy-2-ethylhexylcarbonat oder Kombinationen von zwei oder mehreren davon.

12. Polymerisierbares Dentalmaterial nach einem der Ansprüche 1 bis 11, **dadurch gekennzeichnet, dass** Komponente A
a) 10 bis 85 Gew.% wenigstens eines Reaktiv-Pastenbildners der Komponente a) und/oder b) enthält, und
b) 0,5 bis 20 Gew. % der Komponente c),
wobei die Prozentangaben sich auf die Gesamtmasse der Komponente A beziehen, und dass Komponente B
c) 20 bis 85 Gew. % an Komponente d),
d) 1 bis 100 ppm an Komponente e) und
e) 0,01 bis 1 Gew. % an Komponente f) enthält,
wobei die Prozentangaben sich auf die Gesamtmasse der Komponente B beziehen.

13. Polymerisierbares Dentalmaterial nach einem der Ansprüche 1 bis 12, **dadurch gekennzeichnet, dass** das Dentalmaterial in Komponenten A und/oder B einen oder mehrere Zusatzstoffe i) enthalten, vorzugsweise Puffersalze, Wasserfänger, Metallfänger, Metallkomplexbildner, weitere Pastenbildner, Tenside, Wirkstoffe, optische Abtastung ermöglichende Substanzen, Geschmacks- und/oder Geruchsstoffe, Diagnostik ermöglichende Substanzen, Zahnsubstanz ätzende und/oder adhäsiv wirkende Substanzen, Fluoridisierungsmittel, Bleichsubstanzen, Desensibilisierungsmittel, Haftverbundvermittler, Farbstoffe, Farbpigmente, Indikatoren, weitere von Komponenten c), e), f) und h) unterschiedliche Initiatoren oder Initiatorkomponenten, Stabilisatoren, Polymerisationsinhibitoren, Thixotropiehilfsmittel sowie antibakterielle Substanzen oder Kombinationen von zwei oder mehreren davon.

14. Gehärtetes Dentalmaterial, erhältlich durch Vermischen der Komponenten A und B gemäß einem der Ansprüche 1 bis 13 im Verhältnis 1: 20 bis 1: 1 und durch Polymerisation des dadurch erhaltenen polymerisierbaren Dentalmaterials.

15. Verwendung des polymerisierbaren Dentalmaterials oder des polymerisierten Dentalmaterials nach einem der vorhergehenden Ansprüche als Befestigungsmaterial, Bondingmaterial, Stumpfaufbaumaterial, zahn-technischen Werkstoff zur Herstellung von Inlays, von Onlays, von Verblendschalen und von künstlichen Zähnen, als Modellmaterial, Dentalzement sowie als provisorisches und permanentes Kronen- und Brückenmaterial, insbesondere zur Herstellung von Kronen- und Brückenmaterial oder zum Einsatz als Dentalzement oder Verwendung des polymerisierbaren Dentalmaterials oder des polymerisierten Dentalmaterials nach einem der vorhergehenden Ansprüche zur Herstellung eines Füllungsmaterials, eines Fissuren-Versiegelungsmaterials oder eines Wurzelkanal-Versiegelungsmaterials.

## Claims

1. A polymerizable dental material containing at least one paste-like component A and at least one paste-like component B, whereby component A contains at least one initiator of the radical polymerization c) selected from the group of barbituric acid derivatives and/or malonyl sulfamides and whereby component B contains an organic compound d) comprising at least one acrylic acid ester and/or methacrylic acid ester residues, and at least one metal compound e), and at least one halide or pseudo halide compound f),wherein ingredient e) is a metal compound selected from the group comprising salts of the metals of the third and fourth main group and the first to eighth secondary group of the periodic table of the elements, including the lanthanides and/or metal complexes, wherein ingredient f) is a halide or pseudo-halide with a metal cations of metals of the first and the second main group of the periodic table, ammonium or phosphonium cations wherein component A as reactive paste-forming agent has at least one organic compound a) derived from maleic acid and/or fumaric acid that has no additional ethylenically unsaturated groups in addition to the groups derived from maleic acid and/or fumaric acid, and/or at least one compound b) comprising at least one allyl and/or methallyl residue and if appropriate, units derived from maleic acid and/or fumaric acid that do not contain any ethylenically unsaturated groups in addition to the ethylenically unsaturated groups cited above, **characterized in that** component A as reactive paste-forming agent contains at least one organic compound a) derived from maleic acid and/or fumaric acid that has no ethylenically unsaturated groups other than those groups derived from maleic acid and/or fumaric acid, and/or at least one compound b) comprising at least one allyl and/or methallyl residue and, if appropriate, units derived from maleic acid and/or fumaric acid that do not contain any ethylenically unsaturated groups in addition to the ethylenically unsaturated groups cited above.

2. A polymerizable dental material as recited in Claim 1,
wherein ingredient a) contains at least one organic compound that contains maleic acid diester and/or fumaric acid diester residues or that contains maleic acid diamides and/or fumaric acid diamides residues, in particular an organic compound represented by the Formulas Ia, Ib, IIa, IIb and in particular, the Formulas IIc or IId: 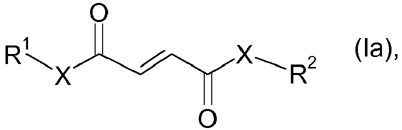 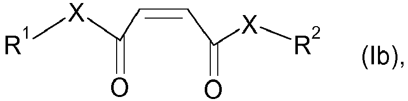 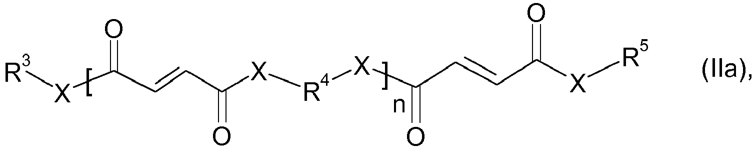 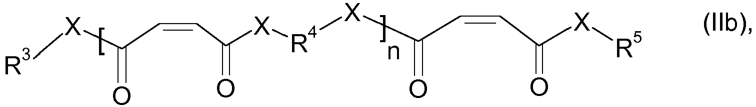 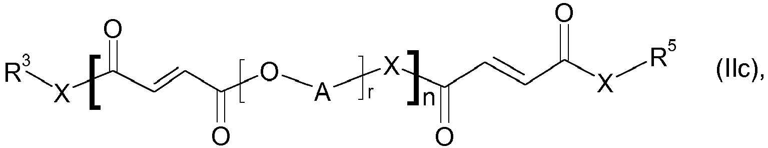 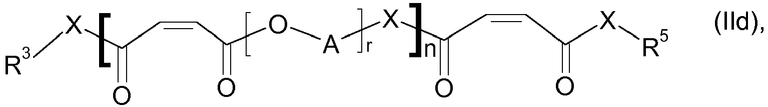
in which X is oxygen or -NR⁶- is a group,
R¹, R², R³ and R⁵, independent of each other, mean alkyl, cycloalkyl, alkyl cycloalkyl, aryl, alkyl aryl, aralkyl or heterocyclyl, which have, if appropriate, one or more substituents,
R⁴ means alkylene, alkylene glycol ether, cycloalkylene, alkyl cycloalkylene, arylene, alkyl arylene, aralkylene or heterocyclylene that has, if appropriate, one or more substituents,
A = CH₂-CH₂, CH₂-CH(CH₃) or CH₂-CH₂-CH₂-CH₂,
R⁶ is hydrogen, alkyl, cycloalkyl, alkyl cycloalkyl, aryl, alkyl aryl, aralkyl or heterocyclyl,
n is a whole number from 1 to 50, and
r is a whole number from 1 to 100.

3. A polymerizable dental material as recited in Claim 2,
wherein one or more residues R¹ to R⁵ have substituents with acidic function, in particular, phosphoric acid groups, phosphonic acid groups, sulfonic acid groups and/or carboxylic acid groups and their anhydrides, or that one or more of the residuals R¹ to R⁵ have substituents with acidic function and hydroxyl groups as substituents, or that mixtures of acid-functionalized and hydroxy-functionalized compounds represented by the Formulas Ia, Ib, IIa, IIb, IIc and/or lid are used as ingredient a).

4. A polymerizable dental material as recited in one of Claims 1 through 3, wherein ingredient b) is a compound containing allyl ether and/or methallyl ether residues and which does not contain any other ethylenically unsaturated groups, in particular, a compound represented by Formula III
R⁷-[-Y-R⁸]ₘ (III),
in which R⁷ is an m-valent residue that perhaps has one or several substituents,
R⁸ is a -CH₂-CR⁹=CH₂ group,
R⁹ means hydrogen or methyl,
m is a whole number from 1 to 12 ist, and
Y is selected from the group of covalent bonds or a bivalent residue.

5. A polymerizable dental material as recited in Claim 4,
wherein the residue R⁷ has substituents with acidic function, in particular phosphoric acid groups, phosphonic acid groups, sulfonic acid groups and/or carboxylic acid groups and their anhydrides, or that the residue, R⁷ has substituents with acidic function and hydroxyl groups as substituents, or that mixtures of acid-functionalized and hydroxy-functionalized compounds represented by Formula III are used as ingredient b).

6. A polymerizable dental material as recited in one of Claims 1 to 5,
wherein ingredient c) is selected from the group of compounds represented by Formula IV, V or VI 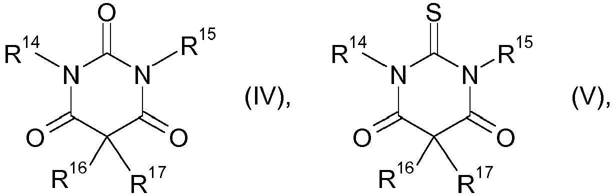 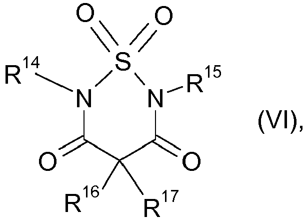
in which R¹⁶ and R¹⁷, independent of each, other mean hydrogen, alkyl, alkenyl, cycloalkyl, aryl, aralkyl and heterocyclyl, provided that at least one of the residues R¹⁶ or R¹⁷ means hydrogen, and
R¹⁴ and R¹⁵, independent of each, other mean hydrogen, alkyl, alkenyl, cycloalkyl, aryl, aralkyl and heterocyclyl, in particular, from the group of barbituric acid derivatives represented by Formula IV, in which R¹⁴ and/or R¹⁶ or R¹⁷, independent of each other, mean alkyl, cycloalkyl, aryl or aralkyl.

7. A polymerizable dental material as recited in one of Claims 1 through 6, wherein ingredient d) is an organic compound represented by the Formula VII: 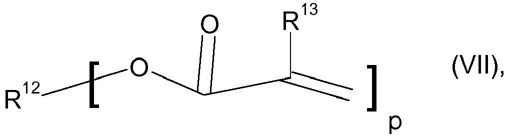
in which R¹² is a p-valent organic residue, that perhaps has one or more substituents,
R¹³ means hydrogen or methyl, and
p is a whole number from 1 to 12.

8. A polymerizable dental material as recited in one of Claims 1 through 7, wherein ingredient e) comprises anions derived from carboxylic acids, preferably from aliphatic or aromatic carboxylic acids, in particular acrylic acid or methacrylic acid, particularly preferred, salts of copper, iron, tin, chromium manganese, cobalt, zinc, nickel, the rare earths' and aluminum, or the complexes of metals of the third and fourth main group and the first to eighth secondary group of the periodic table of the elements, including lanthanides with ligands derived from acetylacetonate, particularly preferred, complexes of copper, iron, tin, chromium, manganese, cobalt, zinc, nickel, the rare earths' and aluminum, and in which most particularly preferred, ingredient e) is a copper compound, in particular, copper acetylacetonate, copper naphthenate, copper methacrylate, copper acrylate, copper acetate, copper oleate, copper ethylhexanoate or copper cyclohexyl butyrate.

9. A polymerizable dental material as recited in one of Claims 1 through 8, wherein ingredient f) comprises ammonium or phosphonium cations with organic residues including the hydro-halides of amines, most particularly preferred a chloride, bromide or iodide that has as a cation a metal cation, in particular, a lithium cation or a sodium cation or an ammonium cation or a phosphonium cation or a thiocyanate, isothiocyanate, cyanate or isocyanate, that has a lithium cation or a sodium cation or an ammonium cation or a phosphonium cation as cation or a hydro-halide of a tertiary amine and in which ingredient f) is most particularly preferred, selected from lithium, ammonium or phosphonium halides or their hydro-halides.

10. A polymerizable dental material as recited in one of Claims 1 through 9, wherein component A and/or B additionally contains at least one filler g) that is present preferably in component A in a quantity of 0 to 80% by weight und in component B in a quantity of 10 to 90% by weight, relative to the total mass of the respective component.

11. A polymerizable dental material as recited in one of Claims 1 to 10,
wherein component A and/or component B additionally contains at least one organic peroxide h), that is preferably present in an amount of up to 5% by weight relative to the total mass of components A and/or B, whereby ingredient h) is particularly preferably, selected from the group of carboxylic acids and/or carbonic acid peroxyesters and/or perketals, most particularly preferred from the group of tert-butylperoxy-3,5,5-trimethyl hexanoate, tert-butylperoxybenzoate, tert-butylperoxy-2-ethylhexylcarbonate or combinations of two or more of such.

12. A polymerizable dental material as recited in one of Claims 1 through 12, wherein component A contains
- 10 to 85 % by weight of at least one reactive paste-forming agent of component a) and/or b), and
- 0.5 to 20% by weight of component c),
in which the percentage information relates to the total mass of component A, and that component B contains
- 20 to 85 % by weight of component d),
- 1 to 100 ppm of component e) and
- 0.01 to 1% by weight of component f),
in which the percentage information relates to the total mass of component B.

13. A polymerizable dental material as recited in one of Claims 1 through 13, wherein the dental material in component A and/or B contains one or more additives i), preferably buffer salts, water scavengers, metal scavengers, metal complex-forming agents, additional paste-forming agents, tensides, active ingredients, substances making optical scanning possible, flavoring substances and/or odorants, substances making diagnostics possible, tooth substance etching and/or substances with adhesive-action, fluoridation agents, bleaching substances, desensitizing agents, composite adhesive agents, colorants, pigments, indicators, additional components c), e), f) and h) various indicators or indicator components, stabilizers, polymerization inhibitors, thixotropy aids and antibacterial substances, or combinations of two or more of such.

14. A hardened dental material obtainable by mixing components A and B as recited in one of the Claims 1 through 13 in a proportion of 1: 20 to 1: 1 and by polymerization of the thereby obtained polymerizable dental material.

15. Use of the polymerizable dental material or the polymerized dental material as recited in one of the preceding claims as fastening material, bonding material, stump-build-up material, dental material for producing inlays, onlays, cup liners and artificial teeth, as model material, dental cement and as temporary and permanent crown and bridge material, in particular for the production of crowns and bridge material or for use as dental cement or use of the polymerizable dental material or the polymerized dental material according to any of the previous claims for production of a filling material, of a fissure sealing material or of a root canal sealing material.

## Revendications

1. Matériau dentaire polymérisable, contenant au moins un constituant pâteux A et au moins un constituant pâteux B, le constituant A contenant au moins un initiateur de polymérisation radicalaire c) choisi dans le groupe des dérivés de l'acide barbiturique et/ou des malonylsulfamides, et le constituant B contenant au moins un composé organique d) qui comprend des résidus d'ester d'acide acrylique et/ou d'ester d'acide méthacrylique, au moins un composé métallique e) et au moins un composé d'halogénure et/ou de pseudohalogénure f), l'ingrédient e) étant un composé métallique choisi dans le groupe des sels de métaux appartenant aux groupes 3 à 12 du tableau périodique des éléments y compris les lanthanides et/ou les complexes de métaux, l'ingrédient f) étant un halogénure ou pseudohalogénure de cations métalliques de métaux appartenant aux groupes 1 et 2 du tableau périodique des éléments, de cations d'ammonium ou de phosphonium, **caractérisé en ce que** le constituant A, en tant qu'agent formant une pâte par voie réactive, contient au moins un composé organique a) qui est dérivé d'acide maléique et/ou d'acide fumarique et qui ne comporte, outre les groupes dérivés d'acide maléique et/ou d'acide fumarique, pas d'autres groupes éthyléniquement insaturés, et/ou au moins un composé b) qui comprend au moins un résidu d'allyle et/ou de méthallyle et, éventuellement, des unités dérivés d'acide maléique et/ou d'acide fumarique et qui ne comporte, outre les groupes éthyléniquement insaturés précités, pas d'autres groupes éthyléniquement insaturés.

2. Matériau dentaire polymérisable selon la revendication 1, **caractérisé en ce que** l'ingrédient a) contient au moins un composé organique comportant des résidus de diester d'acide maléique et/ou de diester d'acide fumarique ou des résidus de diamide d'acide maléique et/ou de diamide d'acide fumarique, s'agissant notamment d'un composé organique répondant aux formules Ia, Ib, IIa, IIb, et notamment aux formules IIc ou IId 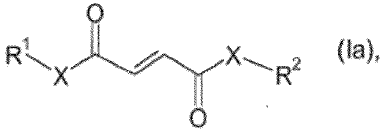 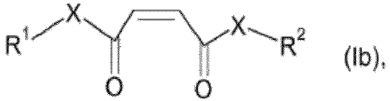 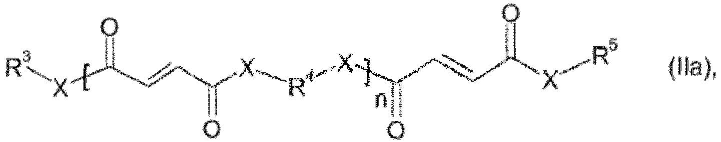 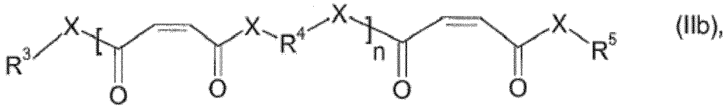 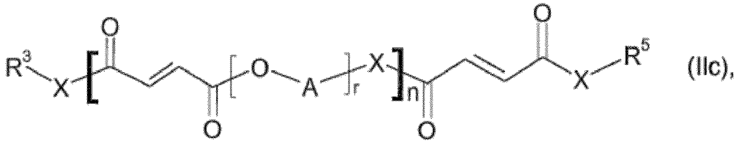 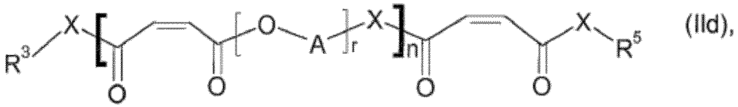
dans lesquelles X représente oxygène ou un groupe -NR⁶-,
R¹, R², R³ et R⁵ représentent, indépendamment l'un de l'autre, alkyle, cycloalkyle, alkylcycloalkyle, aryle, alkylaryle, aralkyle ou hétérocyclyle lesquels sont éventuellement pourvus d'un ou de plusieurs substituants,
R⁴ représente alkylène, alkylèneglycoléther, cycloalkylène, alkylcycloalkylène, arylène, alkylarylène, aralkylène ou heterocyclylène, éventuellement pourvu d'un ou de plusieurs substituants,
A représente CH₂-CH₂, CH₂-CH (CH₃) ou CH₂-CH₂-CH₂-CH₂,
R⁶ représente hydrogène, alkyle, cycloalkyle, alkylcycloalkyle, aryle, alkylaryle, aralkyle ou hétérocyclyle,
n étant un nombre entier compris entre 1 et 50, et
r étant un nombre entier compris entre 1 et 100.

3. Matériau dentaire polymérisable selon la revendication 2, **caractérisé en ce qu'**un ou plusieurs des résidus R¹ à R⁵ sont pourvus de substituants comportant des fonctions acides, s'agissant notamment de groupes d'acide phosphorique, groupes d'acide phosphoreux, groupes d'acide sulfonique et/ou de groupes d'acide carboxylique ainsi que de leurs anhydrides, ou qu'un ou plusieurs des résidus R¹ à R⁵ sont pourvus de substituants comportant des fonctions acides et des groupes hydroxyle en tant que substituants, ou que l'on met en oeuvre, en tant qu'ingrédient a), des mélanges de composés pourvus de fonctions acides et hydroxyles répondant aux formules Ia, Ib, IIa, IIb, IIc et/ou IId.

4. Matériau dentaire polymérisable selon l'une des revendications 1 à 3, **caractérisé en ce que** l'ingrédient b) est un composé qui contient des résidus d'éther allylique et/ou d'éther méthallylique et qui ne comporte, en outre, pas d'autres groupes éthyléniquement insaturés, s'agissant notamment d'un composé répondant à la formule III
R⁷-[-Y-R⁸]ₘ (III),
dans laquelle R⁷ représente un résidu m-valent comportant éventuellement un ou plusieurs substituants,
R⁸ représente un groupe -CH₂-CR⁹=CH₂,
R⁹ représente hydrogène ou méthyle,
m est un nombre entier compris entre 1 et 12, et
Y est choisi dans le groupe d'une liaison covalente ou d'un résidu divalent.

5. Matériau dentaire polymérisable selon la revendication 4, **caractérisé en ce que** le résidus R⁷ est pourvu de substituants comportant des fonctions acides, s'agissant notamment de groupes d'acide phosphorique, groupes d'acide phosphoreux, groupes d'acide sulfonique et/ou de groupes d'acide carboxylique ainsi que de leurs anhydrides, ou que le résidu R⁷ est pourvu de substituants comportant des fonctions acides et de groupes hydroxyle en tant que substituants, ou que l'on met en oeuvre, en tant qu'ingrédient b), des mélanges de composés pourvus de fonctions acides et hydroxyles répondant à la formule III.

6. Matériau dentaire polymérisable selon l'une des revendications 1 à 5, **caractérisé en ce que** l'ingrédient c) est choisi dans le groupe des composés répondant aux formules IV, V ou VI 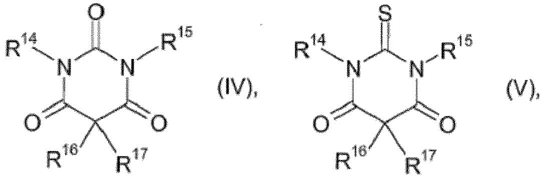 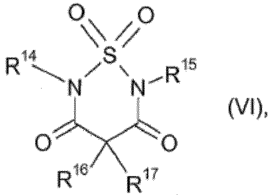
dans lesquelles R¹⁶ et R¹⁷ représentent, indépendamment l'un de l'autre, hydrogène, alkyle, alcényle, cycloalkyle, aryle, aralkyle et hétérocyclyle, à condition qu'au moins un des résidus R¹⁶ ou R¹⁷ représente hydrogène, et
R¹⁴ et R¹⁵ représentent, indépendamment l'un de l'autre, hydrogène, alkyle, alcényle, cycloalkyle, aryle, aralkyle et hétérocyclyle, notamment dans le groupe des dérivés d'acide barbiturique répondant à la formule IV, dans laquelle R¹⁴ et/ou R¹⁶ ou R¹⁷ représentent, indépendamment l'un de l'autre, alkyle, cycloalkyle, aryle ou aralkyle.

7. Matériau dentaire polymérisable selon l'une des revendications 1 à 6, **caractérisé en ce que** l'ingrédient d) est un composé organique répondant à la formule VII, 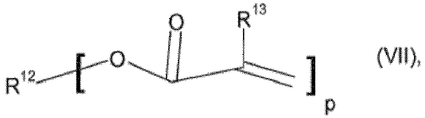 dans laquelle R¹² est un résidu organique p-valent pourvu éventuellement d'un ou de plusieurs substituants, R¹³ représente hydrogène ou méthyle, et p est un nombre entier compris entre 1 et 12.

8. Matériau dentaire polymérisable selon l'une des revendications 1 à 7, **caractérisé en ce que** l'ingrédient e) comprend des anions dérivés d'acides carboxyliques, notamment d'acide acrylique ou d'acide méthacrylique, s'agissant avec une préférence particulière de sels de cuivre, de fer, d'étain, de chrome, de manganèse, de cobalt, de zinc, de nickel, de terres rares et de l'aluminium, ou de complexes de métaux des groupes 3 à 12 du tableau périodique des éléments, y compris les lanthanides, avec des ligands dérivés d'acétylacétonate, s'agissant avec une préférence particulière de complexes de cuivre, de fer, d'étain, de chrome, de manganèse, de cobalt, de zinc, de nickel, de terres rares et d'aluminium, et l'ingrédient e) étant, avec une préférence toute particulière, un composé de cuivre, s'agissant notamment d'acétylacétonate de cuivre, de naphténate de cuivre, de méthacrylate de cuivre, d'acrylate de cuivre, d'oléate de cuivre, d'éthylhexanoate de cuivre ou de cyclohexylbutyrate de cuivre.

9. Matériau dentaire polymérisable selon l'une des revendications 1 à 8, **caractérisé en ce que** l'ingrédient f) comprend des cations d'ammonium ou de phosphonium pourvus de résidus organiques, y compris les hydrohalogénures d'amines, s'agissant avec une préférence toute particulière d'un chlorure, bromure ou iodure comportant, en tant que cation, un cation métallique, notamment un cation de lithium ou un cation de sodium ou un cation d'ammonium ou un cation de phosphonium, ou bien d'un thiocyanate, isothiocyanate, cyanate ou isocyanate comportant, en tant que cation, un cation de lithium ou un cation de sodium ou un cation d'ammonium ou un cation de phosphonium, ou bien d'un hydrohalogénure d'une amine tertiaire, et l'ingrédient f) étant avec une préférence toute particulière choisi parmi les halogénures de lithium, d'ammonium ou de phosphonium ou les hydrohalogénures correspondants.

10. Matériau dentaire polymérisable selon l'une des revendications 1 à 9, **caractérisé en ce que** le constituant A et/ou B contient en outre au moins une charge g) qui est présente, de préférence, au sein du constituant A en une quantité comprise entre 0 et 80 % en poids, et au sein du constituant B en une quantité comprise entre 10 et 90 % en poids, par rapport à la masse totale du constituant concerné.

11. Matériau dentaire selon l'une des revendications 1 bis 10, **caractérisé en ce que** le constituant A et/ou B contient en outre au moins un peroxyde organique h) qui est préférentiellement présent en une quantité pouvant atteindre jusqu'à 5 % en poids, par rapport à la masse totale du constituant A et/ou B, le constituant h) étant choisi, avec une préférence particulière, dans le groupe des peroxyesters d'acide carboxylique et/ou d'acide carbonique et/ou des percétals, avec une préférence toute particulière dans le groupe de tert.-butylperoxy-3,5,5-triméthylhexanoate, tert.-butylperoxybenzoate, tert.-butylperoxy-2-éthylhexylcarbonate ou de combinaison de deux ou de plusieurs de ceux-ci.

12. Matériau dentaire polymérisable selon l'une des revendications 1 à 11, **caractérisé en ce que** le constituant A contient
a) 10 à 85 % en poids d'au moins un agent formant une pâte par voie réactive appartenant aux constituants a) et/ou b), et
b) 0,5 à 20 % en poids du constituant c) les pourcentages étant exprimés par rapport à la masse totale du constituant A, et que le constituant B contient
c) 20 à 85 % en poids du constituant d),
d) 1 à 100 ppm du constituant e), et
e) 0,01 à 1 % en poids du constituant f),
les pourcentages étant exprimés par rapport à la masse totale du constituant B.

13. Matériau dentaire polymérisable selon l'une des revendications 1 à 12, **caractérisé en ce que** le matériau dentaire contient, dans les constituants A et/ou B, un ou plusieurs additifs i), s'agissant préférentiellement sels de tampon, d'absorbants d'humidité, d'absorbants de métaux, d'agents de complexation de métaux, d'autres agents formant une pâte, d'agents tensioactifs, de principes actifs, de substances permettant la mise en oeuvre d'un balayage optique, d'arômes et/ou de parfums, de substances permettant la mise en oeuvre de démarches diagnostiques, de substances ayant un effet corrosif sur la matière des dents et/ou ayant un effet adhésif, d'agent de fluoration, d'agents blanchissants, d'agents de désensibilisation, d'agents de pontage pour systèmes d'adhérence composites, de colorants, de pigments colorés, d'indicateurs, d'initiateurs ou constituants d'initiateurs différents des constituants c), e), f) et h), d'agents stabilisateurs, d'inhibiteurs de polymérisation, d'agents auxiliaires de thixotropie ainsi que de substances antibactériennes ou de combinaisons de deux ou de plusieurs de ceux-ci.

14. Matériau dentaire durci, pouvant être obtenu en mélangeant les constituants 1A et B selon l'une des revendications 1 à 13 dans un rapport compris entre 1 : 20 et 1 : 1 et par polymérisation du matériau dentaire polymérisable ainsi obtenu.

15. Utilisation du matériau dentaire polymérisable ou du matériau dentaire polymérisé selon l'une des revendications précédentes en tant que matériau de fixation, matériau de liaison, matériau de construction de moignons, matériau dentaire technique destiné à fabriquer des inlays, des onlays, des facettes et des dents artificielles, en tant que matériau de moulage, ciment dentaire ainsi que matériau provisoire ou définitif pour couronnes et bridges, plus particulièrement pour fabriquer du matériau dentaire pour couronnes et bridges, ou pour une mise en oeuvre en tant que ciment dentaire, ou bien utilisation du matériau dentaire polymérisable ou du matériau dentaire polymérisé selon l'une des revendications précédentes pour fabriquer un matériau de restauration, un matériau d'obturation de fissures ou un matériau d'obturation canalaire.
